# EUROPEAN PATENT APPLICATION

(11) **EP 4 417 690 A1**
(43) Date of publication of application: **21.08.2024**
(21) Application number: 22947559.5
(22) Date of filing: 29.07.2022
(51) Int. Cl.: C12N 9/10, C12N 15/54, C12N 15/70, C12P 13/00, C12P 17/12

(54) **TRANSAMINASE MUTANT AND PREPARATION METHOD FOR CHIRAL AMINE COMPOUND**

(30) Priority: 21.06.2022 CN 202210707289
(71) Applicant: Asymchem Life Science (Tianjin) Co., Ltd, Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); MA, Yulei, Tianjin 300457 (CN); MU, Huiyan, Tianjin 300457 (CN); XUAN, Meijuan, Tianjin 300457 (CN); ZHANG, Yanqing, Tianjin 300457 (CN); ZHANG, Renkuan, Tianjin 300457 (CN)
(74) Representative: Berggren Oy
(86) International application number: PCT/CN2022/109140
(87) International publication number: WO 2023/245815

(57) **Abstract**

The present disclosure provides a transaminase mutant and a method for preparing a chiral amine compound. Herein, the transaminase mutant includes: (a) a protein having an amino acid sequence as shown in SEQ ID NO: 1; or (b) a protein having an amino acid sequence as shown in SEQ ID NO: 2 or SEQ ID NO: 3; or (c) a protein that undergoes an amino acid mutation in at least one of the following sites of the amino acid sequence in (b): Y89, L380, N86, Y85, T91, P83, K90, S417, S424, F301, G164, T452 and the like, and has a transaminase function; or (d) a protein having more than 80% of the homology with the amino acid sequence defined in any one of (a), (b) or (c) and having a transaminase function.

## Description

### Cross-Reference to Related Application

The present disclosure is based upon and claims priority to Chinese application No. 202210707289.5 filed on June 21, 2022, the disclosure of which is hereby incorporated by reference in its entirety.

### Technical Field

The present disclosure relates to the field of enzyme catalysis, in particular to a transaminase mutant and a method for preparing a chiral amine compound.

### Background

Chiral amines refer to a class of compounds that contain amino groups in the chiral center of small-molecular compounds, which are structural units of many important bioactive molecules and key intermediates in the synthesis of many chiral drugs. At present, the preparation of the chiral amines is mainly achieved by chemical methods, biological separation methods, and biological asymmetric synthesis methods. The chemical methods have the disadvantages, such as long reaction routes, harsh conditions, use of toxic transition metal catalysts, and low product stereoselectivity; and the theoretical maximum yield of the biological separation methods is only 50%, so there are certain limitations in scale-up production.

The asymmetric synthesis methods catalyzed by transaminases already become a preferred method for synthesizing the chiral amines due to its high theoretical yield, high selectivity, high conversion rate, and mild reaction conditions. However, the disadvantages of most wild-type enzymes are that their substrate range is limited and it is often difficult to accept groups with a carbonyl side larger than a methyl substituent (referred to in the present disclosure as synthetic large sterically hindered chiral amine compounds), which means that there are not many transaminases that may truly be industrially applied. The synthesis of the chiral amine compounds with the high steric hindrance catalyzed by the transaminases is a long-standing challenge.

Modifying the wild-type enzymes by directed evolution to improve its substrate acceptance range, especially in the synthesis of the chiral amine compounds with the high steric hindrance, is a good method to solve this challenge. Two good cases are the directed evolution of the wild-type transaminases from different sources by Codexis Company in the United States, and mutants obtained may be used for efficient catalysis of Sitagliptin [Science, 329 (Jul.16TN.5989), 305-309] and Sacubitril [ACS Catal. 2021, 11 (6), 3762-3770] precursor ketones, to synthesize corresponding chiral amine compounds with the high steric hindrance. The biosynthesis technology research and development center team of Asymchem Life Science Technology (Tianjin) Co., Ltd. screens a transaminase mutant derived from *Chromobacterium violaceum* in previous researches, which may be used to catalyze the synthesis of large sterically hindered chiral amines. Afterwards, in order to further adapt to the requirements of high substrate concentration, low enzyme amount, and extreme environment in the industrial production, Asymchem continues to develop transaminases that may synthesize the chiral amine compounds with the high steric hindrance, as to improve production efficiency, reduce industrial production costs, and reduce industrial three waste emissions.

### Summary

A main purpose of the present disclosure is to provide a transaminase mutant and a method for preparing a chiral amine compound, as to solve the problem of low transaminase activity in existing technologies.

In order to achieve the above purpose, according to a first aspect of the present disclosure, a transaminase mutant is provided, and includes: (a) a protein having an amino acid sequence as shown in SEQ ID NO: 1; or (b) a protein having an amino acid sequence as shown in SEQ ID NO: 2 or SEQ ID NO: 3; or (c) a protein that undergoes an amino acid mutation in at least one of the following sites of the amino acid sequence in (b): Y89, L380, N86, Y85, T91, P83, K90, S417, S424, F301, G164, T452, M180, F449, F320, Y322, D315, A31, L295, V64, H154, F409, T402, T126, F364, A433, L379, D416, N151, H274, I311, V327, R77 or N317, and has a transaminase function; or (d) a protein having more than 80% of the homology with the amino acid sequence defined in any one of (a), (b) or (c) and having a transaminase function.

Further, the amino acid mutation of (c) is each independently selected from the following: S424A or S424F or S424Q or S424P or S424R or S424N or S424V or S424Y or S424E or S424I; L380A; S417A or S417Q or S417F or S417I; Y89A or Y89D or Y89S or Y89H or Y89M or Y89G or Y89F; F409A or F409S or F409Q or F409L or F409H or F409P or F409K or F409G or F409V or F409N; N86M or N86P or N86D or N86A or N86V or N86H; Y85M or Y85F or Y85R; P83C or P83A or P83S or P83G or P83R; T91M or T91A or T91V or T91N or T91I; K90Y or K90G or K90A or K90V or K90S; F301S; G164S; T452S; M180V; F449L; F320H; Y322T; D315V or D315C or D315R or D315M; A31V; L295Q or L295M or L295F or L295Q; V64M or V64A or V64S; H154S; T402K or T402S; T126C; F364L; A433T; L379Aor L379S; D416A; N151A; H274Y; I311F; V327S; R77Q; and N317Y, herein, the letter before the number represents an original amino acid, and the letter after the number represents a mutant amino acid; preferably, in (d), the protein has more than 85%, preferably more than 90%, more preferably more than 95%, and further preferably more than 99% of the homology with the amino acid sequence defined in (a), (b) or (c) and has the transaminase function.

Further, the mutation of the transaminase mutant includes any one of the following amino acid mutations:
W60A; Y89A; N151A; F166A; E168A; V234A; I262A; L379A; L380A; R405A; D416A; S417A; C418A; S424A; V234A+S424A; V234A+L380A; V234A+ L379A; V234A+D416A; V234A+S417A; V234A+Y89A; V234A+N151A; V234A+L379A+L380A; V234A+D416A+S417A; V234A+D416A+S424A; V234A+S417A+ S424A; V234A+L380A+Y89D; V234A+L380A+Y89S; V234A+L380A+Y89H; V234A+L380A+Y89M; V234A+L380A+F409A; V234A+L380A+F409S; V234A+L380A+F409Q; V234A+L380A+F409L; V234A+L380A+S424F; V234A+L380A+S424Q; V234A+L380A+S424P; V234A+L380A+S424R; V234A+L380A+S424N; V234A+L380A+N86M; V234A+L380A+N86P; V234A+L380A+N86D; V234A+L380A+N86A; V234A+L380A+N86V; V234A+L380A+N86H; V234A+L380A+N86M+Y89D; V234A+L380A+N86M+Y89S; V234A+L380A+N86M+Y89G; V234A+L380A+N86M+Y89H; V234A+L380A+N86M+Y89F; V234A+L380A+N86M+Y89A; V234A+L380A+Y89D+F409H; V234A+L380A+Y89D+F409A; V234A+L380A+Y89D+F409P; V234A+L380A+Y89D+F409K; V234A+L380A+Y89D+F409G; V234A+L380A+Y89D+S424A; V234A+L380A+Y89D+S424V; V234A+L380A+Y89D+S424Y; V234A+L380A+Y89D+S424E; V234A+L380A+Y89D+S424Q; V234A+L380A+Y89D+N86H; V234A+L380A+Y89D+N86A; V234A+L380A+Y89D+N86H+Y85M; V234A+L380A+Y89D+N86H+Y85F; V234A+L380A+Y89D+N86H+Y85R; V234A+L380A+Y89D+N86H+Y85M+S417Q; V234A+L380A+Y89D+N86H+Y85M+S417F; V234A+L380A+Y89D+N86H+Y85M+S417I; V234A+L380A+Y89D+N86H+Y85M+S424I; V234A+L380A+Y89D+N86H+Y85M+P83C; V234A+L380A+Y89D+N86H+Y85M+P83A; V234A+L380A+Y89D+N86H+Y85M+P83S; V234A+L380A+Y89D+N86H+Y85M+P83G; V234A+L380A+Y890+N86H+Y85M+T91M; V234A+L380A+Y89D+N86H+Y85M+T91A; V234A+L380A+Y89D+N86H+Y85M+T91V; V234A+L380A+Y89D+N86H+Y85M+T91N; V234A+L380A+Y89D+N86H+Y85M+T91I; V234A+L380A+Y89D+N86H+Y85M+T91 M+P83S; V234A+L380A+Y89D+N86H+Y85M+T91M+P83C; V234A+L380A+Y89D+N86H+Y85M+T91M+P83A; V234A+L380A+Y89D+N86H+Y85M+T91M+P83G; V234A+L380A+Y89D+N86H+Y85M+T91M+P83R; V234A+L380A+Y89D+N86H+Y85M+T91M+P83S+K90Y; V234A+L380A+Y89D+N86H+Y85M+T91M+P83S+K90G; V234A+L380A+Y89D+N86H+Y85M+T91M+P83S+K90A; V234A+L380A+Y89D+N86H+Y85M+T91M+P83S+K90V; V234A+L380A+Y89D+N86H+Y85M+T91 M+P83S+K90S; V234A+L380A+Y89D+N86H+Y85M+T91M+P83S+K90Y+S417I; V234A+L380A+Y89D+N86H+Y85M+T91M+P83S+K90G+S417I; V234A+L380A+Y89D+N86H+Y85M+T91M+P83S+K90Y+S417A; V234C+L380A+Y89D+N86H+Y85M+T91M+P83S+K90Y+S417I; V234A+L380A+Y89D+N86H+Y85M+T91M+P83S+K90Y+S417I+S424A; V234A+L380A+Y89D+N86H+Y85M+T91M+P83S+K90G+S417I+S424A; V234C+L380A+Y89D+N86H+Y85M+T91 M+P83S+K90Y+S417I+S424A; V234A+L380A+Y89D+N86H+Y85M+T91M+P83S+K90Y+S417I+L379S; V234A+L380A+Y89D+N86H+Y85M+T91M+P83S+K90Y+S417I+S424A+F301S; V234A+L380A+Y89D+N86H+Y85M+T91M+P83S+K90G+S417I+S424A+F301S; V234A+L380A+Y89D+N86H+Y85M+T91M+P83S+K90Y+S417I+L379S+H274Y; V234A+L380A+Y89D+N86H+Y85M+T91M+P83S+K90Y+S417I+L379S+A239S; V234A+L380A+Y89D+N86H+Y85M+T91M+P83S+K90Y+S417I+L379S +H274Y+F409L; V234A+L380A+Y89D+N86H+Y85M+T91M+P83S+K90Y+S417I+S424A+G164S; V234A+L380A+Y89D+N86H+Y85M+T91M+P83S+K90Y+S417I+S424A+T452S; V234A+L380A+Y89D+N86H+Y85M+T91M+P83S+K90Y+S4171+S424A+F301S+G164S; V234A+L380A+Y89D+N86H+Y85M+T91 M+P83S+K90G+S417I+S424A+F301S+G164S; V234A+L380A+Y89D+N86H+Y85M+T91 M+P83S+K90Y+S4171+S424A+F301S+G164S+T452S; V234A+L380A+Y89D+N86H+Y85M+T91 M+P83S+K90G+S417I+S424A+F301S+G164S+T452S; V234A+L380A+Y89D+N86H+Y85M+T91M+P83S+K90Y+S417I+S424A+F301S+G164S+T452S+ M180V; V234A+L380A+Y89D+N86H+Y85M+T91M+P83S+K90G+S417I+S424A+F301S+G164S+T452S+ M180V; V234A+L380A+Y89D+N86H+Y85M+T91M+P83S+K90Y+S417I+S424A+F301S+G164S+T452S+ M180V+F449L; V234A+L380A+Y89D+N86H+Y85M+T91M+P83S+K90G+S417I+S424A+F301S+G164S+T452S+ M180V+F449L; V234A+L380A+Y89D+N86H+Y85M+T91M+P83S+K90Y+S417I+S424A+F301S+G164S+T452S+ M180V+F449L+T452S; V234A+L380A+Y89D+N86H+Y85M+T91M+P83S+K90Y+S417I+S424A+F301S+G164S+T452S+ M180V+F449L+F320H; V234A+L380A+Y89D+N86H+Y85M+T91M+P83S+K90Y+S417I+S424A+F301S+G164S+T452S+ M180V+F449L+Y322T; V234A+L380A+Y89D+N86H+Y85M+T91M+P83S+K90Y+S417I+S424A+F301S+G164S+T452S+ M180V+F449L+F320H +Y322T; V234A+L380A+Y89D+N86H+Y85M+T91M+P83S+K90G+S417I+S424A+F301S+G164S+T452S+ M180V+F449L+F320H +Y322T; V234A+L380A+Y89D+N86H+Y85M+T91I+P83S+K90Y+S417I+S424A+F301S+G164S+T452S+M 180V+F449L+F320H+Y322T; V234A+L380A+Y89D+N86H+Y85M+T91I+P83S+K90G+S417I+S424A+F301S+G164S+T452S+M 180V+F449L+F320H+Y322T; V234A+L380A+Y89D+N86H+Y85M+T91I+P83S+K90Y+S417I+S424A+F301S+G164S+T452S+M 180V+F449L+F320H+Y322T+D315V; V234A+L380A+Y89D+N86H+Y85M+T91I+P83S+K90Y+S417I+S424A+F301S+G164S+T452S+M 180V+F449L+F320H+Y322T+I311F; V234A+L380A+Y89D+N86H+Y85M+T91I+P83S+K90Y+S417I+S424A+F301S+G164S+T452S+M 180V+F449L+F320H+Y322T+D315V+A31V; V234A+L380A+Y89D+N86H+Y85M+T91I+P83S+K90Y+S417I+S424A+F301S+G164S+T452S+M 180V+F449L+F320H+Y322T+D315C+A31V; V234A+L380A+Y89D+N86H+Y85M+T91I+P83S+K90Y+S417I+S424A+F301S+G164S+T452S+M 180V+F449L+F320H+Y322T+D315R+A31V; V234A+L380A+Y89D+N86H+Y85M+T91I+P83S+K90Y+S417I+S424A+F301S+G164S+T452S+M 180V+F449L+F320H+Y322T+D315R+A31V+V327S; V234A+L380A+Y89D+N86H+Y85M+T91I+P83S+K90Y+S417I+S424A+F301S+G164S+T452S+M 180V+F449L+F320H+Y322T+D315R+A31V+L295Q; V234A+L380A+Y89D+N86H+Y85M+T91I+P83S+K90Y+S417I+S424A+F301S+G164S+T452S+M 180V+F449L+F320H+Y322T+D315R+A31V+L295M; V234A+L380A+Y89D+N86H+Y85M+T91I+P83S+K90Y+S417I+S424A+F301S+G164S+T452S+M 180V+F449L+F320H+Y322T+D315R+A31V+L295F; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M; V234A+L380A+Y89D+N86H+Y85M+T91I+P83S+K90Y+S417I+S424A+F301S+G164S+T452S+M 180V+F449L+F320H+Y322T+D315R+A31V+L295Q+V64A; V234A+L380A+Y89D+N86H+Y85M+T91I+P83S+K90Y+S417I+S424A+F301S+G164S+T452S+M 180V+F449L+F320H+Y322T+D315R+A31V+L295Q+V64S; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409Q; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409V; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409N; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409N+T402K; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409V+T402S; V234A+L380A+Y89D+N86H+Y85M+T91I+P83S+K90Y+S417I+S424A+F301S+G164S+T452S+M 180V+F449L+F320H+Y322T+D315R+A31V+L295Q+V64M+H154S+F409V+T402S+T126C; V234A+L380A+Y89D+N86H+Y85M+T91I+P83S+K90Y+S417I+S424A+F301S+G164S+T452S+M 180V+F449L+F320H+Y322T+D315M+A31V+L295Q+V64M+H154S+F409V+T402S; V234A+L380A+Y89D+N86H+Y85M+T91I+P83S+K90Y+S417I+S424A+F301S+G164S+T452S+M 180V+F449L+F320H+Y322T+D315V+A31V+L295Q+V64M+H154S+F409V+T402S; V234A+L380A+Y89D+N86H+Y85M+T91 I+P83S+K90G+S417I+S424A+F301S+G164S+T452S+M 180V+F449L+F320H+Y322T+D315R+A31V+L295Q+V64M+H154S+F409V+T402S+T126C; V234A+L380A+Y89D+N86H+Y85M+T91 I+P83S+K90G+S417I+S424A+F301S+G164S+T452S+M 180V+F449L+F320H+Y322T+D315M+A31V+L295Q+V64M+H154S+F409V+T402S; V234A+L380A+Y89D+N86H+Y85M+T91 I+P83S+K90G+S417I+S424A+F301S+G164S+T452S+M 180V+F449L+F320H+Y322T+D315V+A31V+L295Q+V64M+H154S+F409V+T402S; V234A+L380A+Y89D+N86H+Y85M+T91 I+P83S+K90G+S417I+S424A+F301S+G164S+T452S+M 180V+F449L+F320H+Y322T+D315V+A31V+L295Q+V64M+H154S+F409V+T402S+T126C; V234A+L380A+Y89D+N86H+Y85M+T91 I+P83S+K90G+S417I+S424A+F301S+G164S+T452S+M 180V+F449L+F320H+Y322T+D315V+A31V+L295Q+V64M+H154S+F409V+T402S+T126C+F364 L; V234A+L380A+Y89D+N86H+Y85M+T91 I+P83S+K90G+S417I+S424A+F301S+G164S+T452S+M 180V+F449L+F320H+Y322T+D315V+A31V+L295Q+V64M+H154S+F409V+T402S+T126C+F364 L+R77Q; V234A+L380A+Y89D+N86H+Y85M+T91I+P83S+K90G+S417I+S424A+F301S+G164S+T452S+M 180V+F449L+F320H+Y322T+D315V+A31V+L295Q+V64M+H154S+F409V+T402S+T126C+F364 L+N317Y; V234A+L380A+Y89D+N86H+Y85M+T91I+P83S+K90G+S417I+S424A+F301S+G164S+T452S+M 180V+F449L+F320H+Y322T+D315V+A31V+L295Q+V64M+H154S+F409V+T402S+T126C+F364 L+A433T; or V234A+L380A+Y89D+N86H+Y85M+T91I+P83S+K90G+S417I+S424A+F301S+G164S+T452S+M 180V+F449L+F320H+Y322T+D315V+A31V+L295Q+V64MH154S+F409V+T402S+T126C+F364L +N317Y.

In order to achieve the above purpose, according to a second aspect of the present disclosure, a DNA molecule is provided, and the DNA molecule encodes the above transaminase mutant.

In order to achieve the above purpose, according to a third aspect of the present disclosure, a recombinant plasmid is provided, and the recombinant plasmid is linked with the above DNA molecule.

In order to achieve the above purpose, according to a fourth aspect of the present disclosure, a host cell is provided, and the above recombinant plasmid is transformed in the host cell.

Further, the host cell includes a prokaryotic cell; and preferably, the prokaryotic cell includes *Escherichia coli.*

In order to achieve the above purpose, according to a fifth aspect of the present disclosure, a method for preparing a chiral amine compound is provided, and the method includes using the above transaminase mutant under the action of an amino donor, and performing a transamination reaction on a ketone substrate shown in Formula I, to prepare the chiral amine compound,

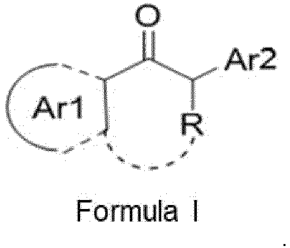

Ar1 is selected from first substituted aryl, first unsubstituted aryl, substituted arylene or unsubstituted arylene, substituted heteroarylene or unsubstituted heteroarylene; Ar2 is selected from second substituted aryl, second unsubstituted aryl, substituted cycloalkyl, unsubstituted cycloalkyl, alkyl or alkylene; R is selected from H, alkyl, alkylene or alkylidene, the number of C atoms of the alkylene or alkylidene is selected from 1-5, and the alkylene or alkylidene includes substituted alkylene or alkylidene or unsubstituted alkylene or alkylidene; while R is selected from the alkylene or alkylidene, the alkylene or alkylidene is linked with Ar1 and/or Ar2 to form a ring; substituents in the first substituted aryl, the substituted arylene, the substituted heteroarylene, the second substituted aryl or the substituted alkylene or alkylidene are each independently selected from halogen, hydroxyl, amino, methyl, ethyl or -CH₂CH₂OH; and heteroatoms in the substituted heteroarylene are selected from N, O or S.

Further, the substituents of the first substituted aryl, the second substituted aryl or the substituted arylene are each independently selected from halogen or -CH₂CH₂OH; preferably, the substituents are each independently located at any one or more of an ortho position, a meta position or a para position of the first substituted aryl, the second substituted aryl or the substituted arylene; and preferably, the halogen is selected from F, Cl or Br.

Further, Ar1 is selected from the first substituted aryl, the substituted arylene, the first unsubstituted aryl or the unsubstituted arylene, Ar2 is selected from the second unsubstituted aryl, R is selected from the unsubstituted alkylene, and the number of C atoms of the unsubstituted alkylene is selected from 1-5; and the unsubstituted alkylene is linked with Ar1 to form a ring.

Further, Ar1 is selected from the unsubstituted heteroarylene, Ar2 is selected from the second substituted aryl, and the substituent of the second substituted aryl is selected from the halogen; R is selected from the substituted alkylene, the substituent of the substituted alkylene is selected from the hydroxyl, and the number of C atoms of the substituted alkylene is selected from 1-5; and the substituted alkylene is linked with Ar1 to form a ring.

Further, Ar1 is selected from the substituted arylene, and the substituent of the substituted arylene is the halogen; Ar2 is selected from the substituted cycloalkyl or the unsubstituted cycloalkyl, and the number of C atoms of the substituted cycloalkyl or the unsubstituted cycloalkyl is selected from 3-8; and R is selected from H.

Further, Ar1 is selected from the first unsubstituted aryl; Ar2 is selected from the second substituted aryl, the second unsubstituted aryl, the substituted cycloalkyl or the unsubstituted cycloalkyl; and R is selected from H, methyl, methylene or methine.

Further, Ar1 is selected from the cycloalkyl or the first substituted aryl, and the substituent is selected from hydroxyl, methyl, ethyl or -CH₂CH₂OH; Ar2 is selected from the second unsubstituted aryl or the alkyl; and R is selected from H, methyl, methylene or methine.

Further, the ketone substrate is selected from

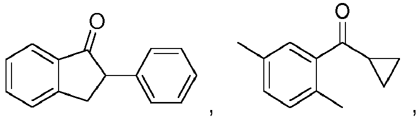

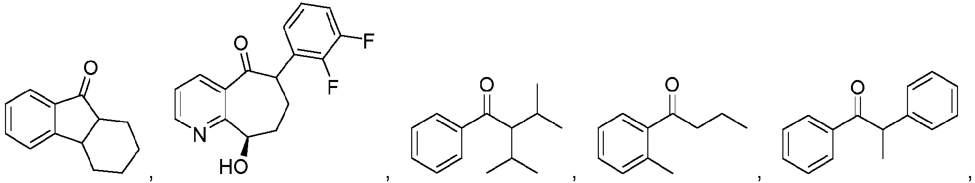

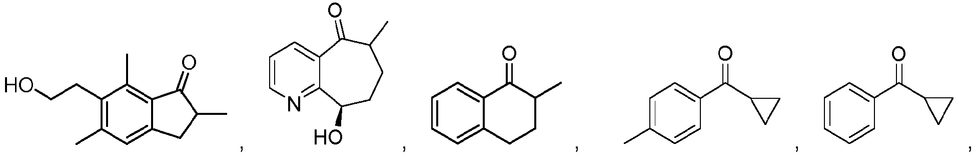

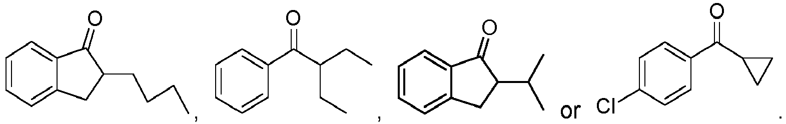

Further, the amino donor includes isopropylamine, isopropylamine hydrochloride, alanine, n-butylamine or aniline.

By applying technical schemes of the present disclosure, the transaminase mutant (SEQ ID NO: 1) derived from *Chromobaterium violaceum* is used as a female parent, and protein engineering modifications such as single point directed mutagenesis, saturation mutagenesis, combination mutagenesis, and error prone polymerase chain reaction (PCR) are performed, to obtain a transaminase mutant with wide substrate spectrum, ability to catalyze the synthesis of large sterically hindered chiral amine compounds, high enzyme activity, and strong tolerance to extreme environments.

### Detailed Description of the Embodiments

It should be noted that, in the case without conflicting, embodiments in the present disclosure and features in the embodiments may be combined with each other. The present disclosure is described in detail below in combination with the embodiments.

As mentioned in the background, the asymmetric synthesis methods catalyzed by transaminases already become a preferred method for synthesizing chiral amines due to its high theoretical yield, high selectivity, high conversion rate, and mild reaction conditions. However, the disadvantages of most wild-type enzymes are that their substrate range is limited, and it is often difficult to synthesize large sterically hindered chiral amine compounds with large sterically hindered ketone substrates as a substrate.

Therefore, in the present disclosure, the inventor attempts to mutate the transaminases and discovers a plurality of key active amino acid sites, thereby the above various transaminase mutants are obtained, which may catalyze the synthesis of the large sterically hindered chiral amine compounds. Therefore, a series of protection schemes are proposed in the present disclosure.

In a first typical implementation mode of the present disclosure, a transaminase mutant is provided, and includes (a) a protein having an amino acid sequence as shown in SEQ ID NO: 1; or (b) a protein having an amino acid sequence as shown in SEQ ID NO: 2 or SEQ ID NO: 3; or (c) a protein that undergoes an amino acid mutation in at least one of the following sites of the amino acid sequence in (b): Y89, L380, N86, Y85, T91, P83, K90, S417, S424, F301, G164, T452, M180, F449, F320, Y322, D315, A31, L295, V64, H154, F409, T402, T126, F364, A433, L379, D416, N151, H274, I311, V327, R77 or N317, and has a transaminase function; or (d) a protein having more than 80% of the homology with the amino acid sequence defined in any one of (a), (b) or (c) and having a transaminase function.

The above amino acid sequence as shown in SEQ ID NO: 1 is an amino acid mutant derived from *Chromobaterium violaceum.* By performing computer simulation and molecular biology experiments such as homology modeling, active site simulation, and directed mutagenesis on this amino acid sequence, a key amino acid site V234 is discovered. V is mutated into A to obtain the amino acid sequence as shown in SEQ ID NO: 2, or mutated into C to obtain the amino acid sequence as shown in SEQ ID NO: 3. Based on the V234 mutated amino acids, it is found that active sites such as Y89, L380, N86, Y85, T91, P83, K90, S417, S424, F301, G164, T452, M180, F449, F320, Y322, D315, A31, L295, V64, H154, F409, T402, T126, F364, A433, L379, D416, N151, H274, I311, V327, R77, or N317 also have the significant impact on protein activity. By mutating the above amino acid sites, proteins with transaminase functions and even enhanced transaminase functions may be obtained. For the above proteins obtained, changes may be made in non-key mutation sites and active sites, proteins having more than 80% of the homology with the above amino acid sequence and having the transaminase functions may be obtained.
SEQ ID NO: 1: 
MQKQRTCSQWRELDAAHHLHPFTDTASLNQAGARVMTRGEGVYLWDCEGNKIIDGMAGAW
CVNVGYGRKDFAEAARRQMEELPFYNTAYKTTHPAVVELSSLLAEVTPAGFDRVFYTNSGSESVD
TMIRMVRRYWDVQGKPEKKTLIGRWNGYHGSTIGGASLGGFKEMHEQGDLPIPGMAHIEQPWW
YKHGKDMTPDEFGVVAARWLEEKILEIGADKVAAFVGEPIQGAGGVIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVSAPPLVMTRAEVDEMLAVAERCLEEFEQTL
KARGLA.
SEQ ID NO: 2: 
MQKQRTCSQWRELDAAHHLHPFTDTASLNQAGARVMTRGEGVYLWDCEGNKIIDGMAGAW
CVNVGYGRKDFAEAARRQMEELPFYNTAYKTTHPAVVELSSLLAEVTPAGFDRVFYTNSGSESVD
TMIRMVRRYWDVQGKPEKKTLIGRWNGYHGSTIGGASLGGFKEMHEQGDLPIPGMAHIEQPWW
YKHGKDMTPDEFGVVAARWLEEKILEIGADKVAAFVGEPIQGAGGAIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVSAPPLVMTRAEVDEMLAVAERCLEEFEQTL
KARGLA.
SEQ ID NO: 3:

MQKQRTCSQWRELDAAHHLHPFTDTASLNQAGARVMTRGEGVYLWDCEGNKIIDGMAGAW
CVNVGYGRKDFAEAARRQMEELPFYNTAYKTTHPAVVELSSLLAEVTPAGFDRVFYTNSGSESVD
TMIRMVRRYWDVQGKPEKKTLIGRWNGYHGSTIGGASLGGFKEMHEQGDLPIPGMAHIEQPWW
YKHGKDMTPDEFGVVAARWLEEKILEIGADKVAAFVGEPIQGAGGCIVPPATYWPEIERICRKYDVL
LVADEVICGFGRTGEWFGHQHFGFQPDLFTAAKGLSSGYLPIGAVFVGKRVAEGLIAGGDFNHGFT
YSGHPVCAAVAHANVAALRDEGIVQRVKDDIGPYMQKRWRETFSRFEHVDDVRGVGMLLAFTLVK
NKAKRELFPDFGEIGTLCRDIFFRNNLIMDSCGDHIVSAPPLVMTRAEVDEMLAVAERCLEEFEQTL
KARGLA.

In a preferred embodiment, the amino acid mutation of (c) is each independently selected from the following: S424A or S424F or S424Q or S424P or S424R or S424N or S424V or S424Y or S424E or S424I; L380A; S417A or S417Q or S417F or S417I; Y89A or Y89D or Y89S or Y89H or Y89M or Y89G or Y89F; F409A or F409S or F409Q or F409L or F409H or F409P or F409K or F409G or F409V or F409N; N86M or N86P or N86D or N86A or N86V or N86H; Y85M or Y85F or Y85R; P83C or P83A or P83S or P83G or P83R; T91M or T91A or T91V or T91N or T91I; K90Y or K90G or K90A or K90V or K90S; F301S; G164S; T452S; M180V; F449L; F320H; Y322T; D315V or D315C or D315R or D315M; A31V; L295Q or L295M or L295F or L295Q; V64M or V64A or V64S; H154S; T402K or T402S; T126C; F364L; A433T; L379A or L379S; D416A; N151A; H274Y; I311F; V327S; R77Q; and N317Y, herein, the letter before the number represents an original amino acid, and the letter after the number represents a mutant amino acid; preferably, in (d), the protein has more than 85%, preferably more than 90%, more preferably more than 95%, 96%, 97% or 98%, and further preferably more than 99%, 99.9% of the homology with the amino acid sequence defined in (a), (b) or (c) and has the transaminase function.

In the present disclosure, the applicant continues to explore the above active sites and discovers that the active sites are mutated into different amino acids, and correspondingly the protein activities are also different. Specific mutations may enhance the transaminase activity. By experimental exploration, it is found that the above specific mutations performed in the active sites may obtain proteins with enhanced activity. For amino acid mutation sites of transaminase proteins, flexible selection and combination may be made among the above mutations.

In a preferred embodiment, the mutation of the transaminase mutant includes any one of the following amino acid mutations:
W60A; Y89A; N151A; F166A; E168A; V234A; I262A; L379A; L380A; R405A; D416A; S417A; C418A; S424A; V234A+S424A; V234A+L380A; V234A+ L379A; V234A+D416A; V234A+S417A; V234A+Y89A; V234A+N151A; V234A+L379A+L380A; V234A+D416A+S417A; V234A+D416A+S424A; V234A+S417A+ S424A; V234A+L380A+Y89D; V234A+L380A+Y89S; V234A+L380A+Y89H; V234A+L380A+Y89M; V234A+L380A+F409A; V234A+L380A+F409S; V234A+L380A+F409Q; V234A+L380A+F409L; V234A+L380A+S424F; V234A+L380A+S424Q; V234A+L380A+S424P; V234A+L380A+S424R; V234A+L380A+S424N; V234A+L380A+N86M; V234A+L380A+N86P; V234A+L380A+N86D; V234A+L380A+N86A; V234A+L380A+N86V; V234A+L380A+N86H; V234A+L380A+N86M+Y89D; V234A+L380A+N86M+Y89S; V234A+L380A+N86M+Y89G; V234A+L380A+N86M+Y89H; V234A+L380A+N86M+Y89F; V234A+L380A+N86M+Y89A; V234A+L380A+Y89D+F409H; V234A+L380A+Y89D+F409A; V234A+L380A+Y89D+F409P; V234A+L380A+Y89D+F409K; V234A+L380A+Y89D+F409G; V234A+L380A+Y89D+S424A; V234A+L380A+Y89D+S424V; V234A+L380A+Y89D+S424Y; V234A+L380A+Y89D+S424E; V234A+L380A+Y89D+S424Q; V234A+L380A+Y89D+N86H; V234A+L380A+Y89D+N86A; V234A+L380A+Y89D+N86H+Y85M; V234A+L380A+Y89D+N86H+Y85F; V234A+L380A+Y89D+N86H+Y85R; V234A+L380A+Y89D+N86H+Y85M+S417Q; V234A+L380A+Y89D+N86H+Y85M+S417F; V234A+L380A+Y89D+N86H+Y85M+S417I; V234A+L380A+Y89D+N86H+Y85M+S424I; V234A+L380A+Y89D+N86H+Y85M+P83C; V234A+L380A+Y89D+N86H+Y85M+P83A; V234A+L380A+Y89D+N86H+Y85M+P83S; V234A+L380A+Y89D+N86H+Y85M+P83G; V234A+L380A+Y89D+N86H+Y85M+T91M; V234A+L380A+Y89D+N86H+Y85M+T91A; V234A+L380A+Y89D+N86H+Y85M+T91V; V234A+L380A+Y89D+N86H+Y85M+T91N; V234A+L380A+Y89D+N86H+Y85M+T91I; V234A+L380A+Y89D+N86H+Y85M+T91 M+P83S;
V234A+L380A+Y89D+N86H+Y85M+T91 M+P83C;
V234A+L380A+Y89D+N86H+Y85M+T91 M+P83A;
V234A+L380A+Y89D+N86H+Y85M+T91M+P83G;
V234A+L380A+Y89D+N86H+Y85M+T91M+P83R;
V234A+L380A+Y89D+N86H+Y85M+T91M+P83S+K90Y;
V234A+L380A+Y89D+N86H+Y85M+T91M+P83S+K90G;
V234A+L380A+Y89D+N86H+Y85M+T91M+P83S+K90A;
V234A+L380A+Y89D+N86H+Y85M+T91M+P83S+K90V;
V234A+L380A+Y89D+N86H+Y85M+T91 M+P83S+K90S;
V234A+L380A+Y89D+N86H+Y85M+T91M+P83S+K90Y+S417I;
V234A+L380A+Y89D+N86H+Y85M+T91M+P83S+K90G+S417I;
V234A+L380A+Y89D+N86H+Y85M+T91M+P83S+K90Y+S417A;
V234C+L380A+Y89D+N86H+Y85M+T91M+P83S+K90Y+S417I;
V234A+L380A+Y89D+N86H+Y85M+T91M+P83S+K90Y+S417I+S424A;
V234A+L380A+Y89D+N86H+Y85M+T91 M+P83S+K90G+S417I+S424A;
V234C+L380A+Y89D+N86H+Y85M+T91M+P83S+K90Y+S417I+S424A;
V234A+L380A+Y89D+N86H+Y85M+T91M+P83S+K90Y+S4171+L379S;
V234A+L380A+Y89D+N86H+Y85M+T91M+P83S+K90Y+S4171+S424A+F301S;
V234A+L380A+Y89D+N86H+Y85M+T91M+P83S+K90G+S417I+S424A+F301S;
V234A+L380A+Y89D+N86H+Y85M+T91M+P83S+K90Y+S417I+L379S+H274Y;
V234A+L380A+Y89D+N86H+Y85M+T91M+P83S+K90Y+S417I+L379S+A239S;
V234A+L380A+Y89D+N86H+Y85M+T91M+P83S+K90Y+S417I+L379S +H274Y+F409L;
V234A+L380A+Y89D+N86H+Y85M+T91M+P83S+K90Y+S417I+S424A+G164S;
V234A+L380A+Y89D+N86H+Y85M+T91M+P83S+K90Y+S417I+S424A+T452S;
V234A+L380A+Y89D+N86H+Y85M+T91M+P83S+K90Y+S4171+S424A+F301S+G164S;
V234A+L380A+Y89D+N86H+Y85M+T91M+P83S+K90G+S417I+S424A+F301S+G164S;
V234A+L380A+Y89D+N86H+Y85M+T91M+P83S+K90Y+S4171+S424A+F301S+G164S+T45 2S;
V234A+L380A+Y89D+N86H+Y85M+T91M+P83S+K90G+S417I+S424A+F301S+G164S+T45 2S;
V234A+L380A+Y89D+N86H+Y85M+T91M+P83S+K90Y+S4171+S424A+F301S+G164S+T45 2S+M180V;
V234A+L380A+Y89D+N86H+Y85M+T91M+P83S+K90G+S417I+S424A+F301S+G164S+T45 2S+M180V;
V234A+L380A+Y89D+N86H+Y85M+T91M+P83S+K90Y+S4171+S424A+F301S+G164S+T45 2S+M180V+F449L;
V234A+L380A+Y89D+N86H+Y85M+T91M+P83S+K90G+S417I+S424A+F301S+G164S+T45 2S+M180V+F449L;
V234A+L380A+Y89D+N86H+Y85M+T91M+P83S+K90Y+S4171+S424A+F301S+G164S+T45 2S+M180V+F449L+T452S;
V234A+L380A+Y89D+N86H+Y85M+T91M+P83S+K90Y+S4171+S424A+F301S+G164S+T45 2S+M180V+F449L+F320H;
V234A+L380A+Y89D+N86H+Y85M+T91M+P83S+K90Y+S4171+S424A+F301S+G164S+T45 2S+M180V+F449L+Y322T;
V234A+L380A+Y89D+N86H+Y85M+T91M+P83S+K90Y+S4171+S424A+F301S+G164S+T45 2S+M180V+F449L+F320H +Y322T;
V234A+L380A+Y89D+N86H+Y85M+T91M+P83S+K90G+S417I+S424A+F301S+G164S+T45 2S+M180V+F449L+F320H +Y322T;
V234A+L380A+Y89D+N86H+Y85M+T91I+P83S+K90Y+S417I+S424A+F301S+G164S+T452 S+M180V+F449L+F320H+Y322T;
V234A+L380A+Y89D+N86H+Y85M+T91I+P83S+K90G+S417I+S424A+F301S+G164S+T452 S+M180V+F449L+F320H+Y322T;
V234A+L380A+Y89D+N86H+Y85M+T91I+P83S+K90Y+S417I+S424A+F301S+G164S+T452 S+M180V+F449L+F320H+Y322T+D315V;
V234A+L380A+Y89D+N86H+Y85M+T91I+P83S+K90Y+S417I+S424A+F301S+G164S+T452 S+M180V+F449L+F320H+Y322T+I311F;
V234A+L380A+Y89D+N86H+Y85M+T91I+P83S+K90Y+S417I+S424A+F301S+G164S+T452 S+M180V+F449L+F320H+Y322T+D315V+A31V;
V234A+L380A+Y89D+N86H+Y85M+T91I+P83S+K90Y+S417I+S424A+F301S+G164S+T452 S+M180V+F449L+F320H+Y322T+D315C+A31V;
V234A+L380A+Y89D+N86H+Y85M+T91I+P83S+K90Y+S417I+S424A+F301S+G164S+T452 S+M180V+F449L+F320H+Y322T+D315R+A31V;
V234A+L380A+Y89D+N86H+Y85M+T91I+P83S+K90Y+S417I+S424A+F301S+G164S+T452 S+M180V+F449L+F320H+Y322T+D315R+A31V+V327S;
V234A+L380A+Y89D+N86H+Y85M+T91I+P83S+K90Y+S417I+S424A+F301S+G164S+T452 S+M180V+F449L+F320H+Y322T+D315R+A31V+L295Q;
V234A+L380A+Y89D+N86H+Y85M+T91I+P83S+K90Y+S417I+S424A+F301S+G164S+T452 S+M180V+F449L+F320H+Y322T+D315R+A31V+L295M;
V234A+L380A+Y89D+N86H+Y85M+T91I+P83S+K90Y+S417I+S424A+F301S+G164S+T452 S+M180V+F449L+F320H+Y322T+D315R+A31V+L295F;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64A;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409Q;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409N;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409N+T402K;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V+T402S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V+T402S+T126C;
V234A+L380A+Y89D+N86H+Y85M+T91I+P83S+K90Y+S417I+S424A+F301S+G164S+T452 S+M180V+F449L+F320H+Y322T+D315M+A31V+L295Q+V64M+H154S+F409V+T402S;
V234A+L380A+Y89D+N86H+Y85M+T91I+P83S+K90Y+S417I+S424A+F301S+G164S+T452 S+M180V+F449L+F320H+Y322T+D315V+A31V+L295Q+V64M+H154S+F409V+T402S;
V234A+L380A+Y89D+N86H+Y85M+T91I+P83S+K90G+S417I+S424A+F301S+G164S+T452 S+M180V+F449L+F320H+Y322T+D315R+A31V+L295Q+V64M+H154S+F409V+T402S+T126C;
V234A+L380A+Y89D+N86H+Y85M+T91I+P83S+K90G+S417I+S424A+F301S+G164S+T452 S+M180V+F449L+F320H+Y322T+D315M+A31V+L295Q+V64M+H154S+F409V+T402S;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S+T126C;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S+T126C+ F364L;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S+T126C+ F364L+R77Q;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S+T126C+ F364L+N317Y;
V234A+L380A+Y89D+N86H+Y85M+T91I+P83S+K90G+S417I+S424A+F301S+G164S+T452 S+M180V+F449L+F320H+Y322T+D315V+A31V+L295Q+V64M+H154S+F409V+T402S+T126C+ F364L+A433T; or
V234A+L380A+Y89D+N86H+Y85M+T91I+P83S+K90G+S417I+S424A+F301S+G164S+T452 S+M180V+F449L+F320H+Y322T+D315V+A31V+L295Q+V64MH154S+F409V+T402S+T126C+F 364L+N317Y.

The above amino acid mutations are all experimentally explored in embodiments of the present disclosure, and all have the transaminase activity. Compared to the female parent with the amino acid sequence as shown in SEQ ID NO: 1, it may obtain a transaminase mutant with wide substrate spectrum, ability to catalyze the synthesis of large sterically hindered chiral amine compounds, high enzyme activity, and/or strong tolerance to extreme environments.

In a second typical implementation mode of the present disclosure, a DNA molecule is provided, and the DNA molecule encodes the above transaminase mutant.

In a third typical implementation mode of the present disclosure, a recombinant plasmid is provided, and the recombinant plasmid is linked with the above DNA molecule.

The above DNA may encode the above transaminase mutant and may be linked to the recombinant plasmid to form a circular DNA. The above DNA and recombinant plasmid may be transcribed and translated under the action of RNA polymerase, ribosome, tRNA and the like, to obtain the above transaminase mutant.

In a fourth typical implementation mode of the present disclosure, a host cell is provided, and the above recombinant plasmid is transformed in the host cell.

In a preferred embodiment, the host cell includes a prokaryotic cell; and preferably, the prokaryotic cell includes *Escherichia coli.*

With the above host cell, the recombinant plasmid may be replicated in the host cell, and the DNA molecule carried by the recombinant plasmid may also be transcribed and translated, to obtain a large number of transaminase mutants. The host cell may obtain the transaminase mutants by cell lysis, protein purification, crude enzyme catalysis after breakage or other modes with prior art, which may be used for subsequent catalysis of amine compounds. The host cell is a non-plant derived host cell.

In a fifth typical implementation mode of the present disclosure, a method for preparing a chiral amine compound is provided, and the method includes using the above transaminase mutant under the action of an amino donor, and performing a transamination reaction on a ketone substrate shown in Formula I, to prepare the chiral amine compound; Ar1 is selected from first substituted aryl, first unsubstituted aryl, substituted arylene or unsubstituted arylene, substituted heteroarylene or unsubstituted heteroarylene; Ar2 is selected from second substituted aryl, second unsubstituted aryl, substituted cycloalkyl, unsubstituted cycloalkyl, alkyl or alkylene; R is selected from H, alkyl, alkylene or alkylidene, the number of C atoms of the alkylene or alkylidene is selected from 1-5, and the alkylene or alkylidene includes substituted alkylene or alkylidene or unsubstituted alkylene or alkylidene; while R is selected from the alkylene or alkylidene, the alkylene or alkylidene is linked with Ar1 and/or Ar2 to form a ring; substituents in the first substituted aryl, the substituted arylene, the substituted heteroarylene, the second substituted aryl or the substituted alkylene or alkylidene are each independently selected from halogen, hydroxyl, amino, methyl, ethyl or -CH₂CH₂OH; and heteroatoms in the substituted heteroarylene are selected from N, O or S.

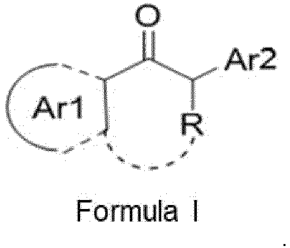

With the above method, under the action of the amino donor provided, the above transaminase mutant may be used to catalyze the ketone compound shown in Formula I, and catalyze a carbonyl into a chiral amino, to obtain the chiral amine compound. The above transaminase mutant is capable of performing chiral catalysis for the synthesis of large sterically hindered chiral amines from the above raw ketone compounds, including ketone compounds with groups larger than a methyl substituent on the side adjacent to the carbonyl. In addition, it may catalyze under industrial production conditions such as high substrate concentration, low enzyme amount, and extreme environments, which may improve production efficiency, reduce industrial production costs, and reduce industrial three waste emissions.

In a preferred embodiment, the substituents of the first substituted aryl, the second substituted aryl or the substituted arylene are each independently selected from halogen or -CH₂CH₂OH; preferably, the substituents are each independently located at any one or more of an ortho position, a meta position or a para position of the first substituted aryl, the second substituted aryl or the substituted arylene; and preferably, the halogen is selected from F, Cl or Br.

In a preferred embodiment, Ar1 is selected from the first substituted aryl, the substituted arylene, the first unsubstituted aryl or the unsubstituted arylene, Ar2 is selected from the second unsubstituted aryl, R is selected from the unsubstituted alkylene, and the number of C atoms of the unsubstituted alkylene is selected from 1-5; and the unsubstituted alkylene is linked with Ar1 to form a ring.

In a preferred embodiment, Ar1 is selected from the unsubstituted heteroarylene, Ar2 is selected from the second substituted aryl, and the substituent of the second substituted aryl is selected from the halogen; R is selected from the substituted alkylene, the substituent of the substituted alkylene is selected from the hydroxyl, and the number of C atoms of the substituted alkylene is selected from 1-5; and the substituted alkylene is linked with Ar1 to form a ring.

In a preferred embodiment, Ar1 is selected from the substituted arylene, and the substituent of the substituted arylene is the halogen; Ar2 is selected from the substituted cycloalkyl or the unsubstituted cycloalkyl, and the number of C atoms of the substituted cycloalkyl or the unsubstituted cycloalkyl is selected from 3-8; and R is selected from H.

In a preferred embodiment, Ar1 is selected from the first unsubstituted aryl; Ar2 is selected from the second substituted aryl, the second unsubstituted aryl, the substituted cycloalkyl or the unsubstituted cycloalkyl; and R is selected from H, methyl, methylene or methine.

In a preferred embodiment, Ar1 is selected from the cycloalkyl or the first substituted aryl, and the substituent is selected from hydroxyl, methyl, ethyl or -CH₂CH₂OH; Ar2 is selected from the second unsubstituted aryl or the alkyl; and R is selected from H, methyl, methylene or methine.

In a preferred embodiment, the ketone substrate is selected from 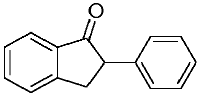 (Substrate 1), 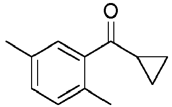 (Substrate 2), 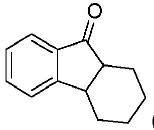 (Substrate 3), 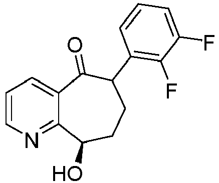 (Substrate 4), 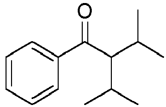 (Substrate 5), 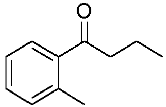 (Substrate 6), 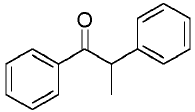 (Substrate 7), 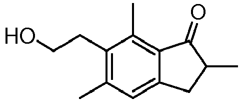 (Substrate 8), 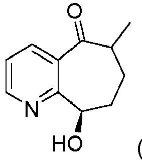 (Substrate 9), 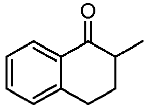 (Substrate 10), 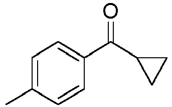 (Substrate 11), 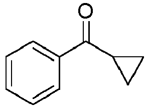 (Substrate 12), 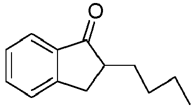 (Substrate 13), 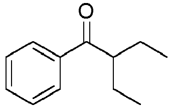 (Substrate 14), 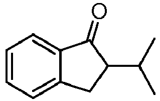 (Substrate 15) or 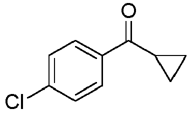 (Substrate 16).

The above ketone substrates have a wide range of substrates, and have the groups larger than the methyl substituent on the side adjacent to the carbonyl. Due to the influence of steric hindrance, it is difficult to use the transaminases in the existing technologies for the chiral catalysis of the above substrates. Moreover, in the existing technologies, even with the transaminases capable of performing the chiral catalysis, it is difficult to achieve large-scale industrial production in high substrate concentrations, low enzyme amounts, extreme environments, and other industrial conditions. With the above transaminase mutants, the efficient chiral catalysis may be performed on the above ketone substrates, and the carbonyl is catalyzed into the chiral amino, to obtain chiral amine compounds with stereoselectivity.

In a preferred embodiment, the amino donor includes but not limited to one or more of isopropylamine, isopropylamine hydrochloride, alanine, n-butylamine or aniline. The amino donor may be flexibly selected or combined among amino donors commonly used in the existing technologies.

In the present disclosure, the inventor performs homologous modeling on the mutants screened according to the three-dimensional structure of the protein (PDB: 4BA5) that is already analyzed, and molecular docking is performed on different large sterically hindered ketone compounds according to the model structure. Docking results are analyzed, 18 residues near the active centers that may affect protein catalytic activity are selected, and these residues include: L59, W60, F88, Y89, N151, Y153, F166, E168, V234, I262, L379, L380, F397, R405, D416, S417, C418, S424. Single point mutations are performed on these residues, all of which are mutated into the alanine. "Alanine scanning" is performed, to observe the impact of these changes on the protein functions.

Site-directed mutagenesis: refers to introduction of a desired change (usually a change that represents a favorable direction) into a target DNA fragment (may be a genome, or a plasmid) by methods such as a polymerase chain reaction (PCR), including addition, deletion, point mutation and the like of bases. The site-directed mutagenesis may rapidly and efficiently improve the characters and representation of a target protein expressed by DNA, and is a very useful means in genetic research work. The introduction method of the site-directed mutagenesis with the whole plasmid PCR is simple and effective, and is a means which is often used at present. A principle thereof is: a pair of primers (forward and reverse) containing the mutation sites, and a template plasmid are annealed to "cyclic extension" with a polymerase, the so-called cyclic extension is that the polymerase extends the primer according to the template, it is returned and terminated at a 5'-end of the primer after a circle, and subjected to the cycle of repeated heating, annealing and extending. This reaction is different from rolling circle amplification, and may not form a plurality of tandem copies. After annealing, extension products of the forward and reverse primers are paired to become a nicked open-circle plasmid, and the extension product is digested with a *Dpn*I enzyme. The original template plasmid is derived from conventional *Escherichia coli,* is modified by dam methylation, is sensitive to *Dpn*I and is cut up, while the plasmid with a mutated sequence synthesized in vitro is not cut up because there is no methylation, and therefore it is successfully transformed in the subsequent transformation, to obtain a clone of a mutant plasmid.

After correct identification through sequencing of site-directed mutant bacteria, the expression of ketone reductase in the mutant bacteria is induced under the condition of overnight induction at 25°C with 0.2 mM isopropyl-β-d-thiogalactoside (IPTG). Then, a crude enzyme is obtained by a method of cell ultrasonication for reaction characteristic detection. After verification of reaction characteristics, the sites that may significantly improve the catalytic properties of the transaminases come from: Y89A, N151A, V234A, L379A, L380A, D416A, S417A, and S424A.

Then, beneficial amino acid sites are combined, to obtain mutants with better traits. A construction method for a double point mutation in combination mutations is the same as that of the single point mutation, and it is constructed with the whole plasmid PCR method. A multi-point mutation with two or more mutation sites is performed simultaneously with overlapping extension PCR amplification, to obtain a mutated gene containing the multi-point mutation. After restriction endonuclease digestion at both ends, it is connected to an expression vector and transformed into *Escherichia coli* cells, then it is spread in an LB culture dish containing 100 µg/mL ampicillin, and cultured overnight at 37°C, to obtain a combination mutant, and it is sequenced and identified. The correct mutant obtained is verified for activity, to obtain a mutant with improved catalytic properties, which may be used as a template for the next round of the saturation mutation. Afterwards, according to a molecular docking model, 20 sites are selected for the saturation mutation in positions of a substrate channel and a key loop region.

The saturation mutation is a method of acquiring a mutant in which an amino acid in a target site is substituted by 19 other amino acids in a short period of time by modifying a coding gene of a target protein. This method is not only a powerful tool for protein directed modification, but also an important means of researching a protein structure-function relationship. The saturation mutation may often obtain more ideal evolutionary bodies than the single point mutation. For these problems that may not be solved by site-directed mutagenesis methods, it is precisely a unique point of saturation mutation methods. The mutant obtained from the saturation mutation is sequenced and identified, and its activity against different substrates and tolerance to high temperatures are tested separately.

After the transaminase mutant with significantly improved activity and tolerance is obtained, random mutations are performed on it with error-prone PCR, to construct a high-quality mutant library. A suitable high-throughput screening method is developed, to screen the library and obtain a mutant with further improved activity.

Error-prone PCR: refers to PCR under error-prone conditions, namely a PCR technology that may easily cause errors in the DNA sequence replicated, it is also known as a mismatch PCR or an error-tendency PCR. Specifically, it refers to a method of inducing a DNA sequence variation in vitro with low fidelity TaqDNA polymerase and changing PCR reaction conditions to reduce the fidelity of DNA replication, increase base mismatches during the synthesis of new DNA chains, and thus result in more point mutations in an amplified product.

The following high-throughput screening method is developed to screen for the error-prone mutant library:
1. Mutant culture: 300 µL of an LB culture medium is added to each well of a 96-well plate, and monoclonal clones from an agar plate are inoculated into the deep-well 96-well plate, and cultured overnight at 37°C and 200 rpm; bacterial solution cultured overnight is transferred to another 96-well plate of which each well is added with 800 µL of the LB culture medium with Qpix, after being cultured at 37°C and 200 rpm for 5 h, and OD600 of the bacterial solution in the 96-well plate reaches 0.6-0.9, IPTG solution is added to the 96-well plate again with Qpix, so that the final concentration of IPTG in the plate is 0.1 mM, and it is induced overnight at 25°C and 200 rpm for about 16 h; and it is centrifuged at 4000 rpm for 5 min, a supernatant is discarded, and whole cells are obtained for use in subsequent reactions .
2. 96-well plate high-throughput screening system:
   8 µL of pyridoxal phosphate (PLP) mother solution (1 mg/mL) and 3.5 µL of of 6 M isopropylamine hydrochloride (20 eq) are supplemented with 0.1 M Tris-Cl 9.0 to 100 µL and mixed uniformly, a mixed sample is divided into a 96-well plate with bacterial sludge in each well, and finally substrate mother solution (0.3 mg of a substrate is dissolved in 15 µL of dimethylsulfoxide (DMSO) which is 15% of the total volume) is added, and mixed uniformly, it is shaken in a shaker at a constant temperature of 50°C and 700 rpm, and reacted for 18 h.

After the completion of the error-prone PCR, the inventor already obtains the mutant with significantly improved activity and tolerance at the trial level. However, in the industrial applications, there are still problems with relatively large enzyme amount and insufficient tolerance. Afterwards, the same mutation method mentioned above is used, and a plurality of rounds of the evolution of the saturation mutations and combination mutations is performed, to obtain a series of transaminase mutants. It is found from results that these transaminase mutants further improve their catalytic efficiency against various ketone substrates and may be used for the synthesis of various chiral amine compounds, especially for the efficient synthesis of the large sterically hindered chiral amine compounds, and the tolerance to the extreme environments is also improved.

The beneficial effects of the present disclosure are further explained in detail below in combination with specific embodiments.

### Embodiment 1

The above method was used, to perform site-specific mutagenesis on the basis of maternal SEQ ID NO: 1. Specific mutation sites were shown in Table 1, and the catalytic activity of the mutant was tested according to the following reaction conditions:
a 1 mL reaction system included 2 mg of Substrate 1 or Substrate 2 or Substrate 3 or Substrate 4, 1 mg of PLP, 2 mg of isopropylamine hydrochloride, 400 µL of crude enzyme solution (prepared from 200 mg of wet bacterial sludge), and pH 8.0 100 mM phosphate buffer, it was reacted at 50°C for 42 h. The above wet bacterial sludge was obtained by centrifugation of *Escherichia coli* fermentation broth of the corresponding mutant, and the crude enzyme solution was obtained by adding pH 8.0 100 mM phosphate buffer to the wet bacterial sludge obtained, homogenizing and breaking walls with ultrasound or homogenizer, and then concentrating.

Detection results were shown in Table 1.

**Table 1:**

| Mutation site | Conversion rate | | | |
|---|---|---|---|---|
| | Substrate 1 | Substrate 2 | Substrate 3 | Substrate 4 |
| Female parent | 0 | 0 | 0 | 0 |
| W60A | 0 | + | 0 | + |
| Y89A | + | 0 | 0 | ++ |
| N151A | ++ | + | + | ++ |
| F166A | 0 | + | + | + |
| E168A | 0 | 0 | 0 | + |
| V234A | ++ | + | ++ | ++ |
| I262A | + | 0 | 0 | 0 |
| L379A | ++ | + | ++ | + |
| L380A | + | ++ | + | ++ |
| R405A | 0 | 0 | + | 0 |
| D416A | ++ | + | + | ++ |
| S417A | + | + | + | + |
| C418A | + | 0 | 0 | + |
| S424A | + | 0 | ++ | ++ |

| | | | | |
|---|---|---|---|---|
| Note: In the above table, 0 represented that the conversion rate was <1%, + represented that the conversion rate was greater than or equal to 1% and less than 5%, and ++ represented that the conversion rate was greater than or equal to 5% and less than or equal to 10%. | | | | |

### Embodiment 2

On the basis of Embodiment 1, a combination mutation was performed, and activity screening was performed on the combination mutation under the same reaction conditions as Embodiment 1. Results were shown in Table 2.

**Table 2:**

| Mutation site | Conversion rate | | | |
|---|---|---|---|---|
| | Substrate 1 | Substrate 2 | Substrate 3 | Substrate 4 |
| V234A | ++ | + | ++ | ++ |
| V234A+S424A | +++ | +++ | + | +++ |
| V234A+L380A | +++ | + | +++ | ++++ |
| V234A+ L379A | + | +++ | + | ++ |
| V234A+D416A | +++ | +++ | +++ | ++ |
| V234A+S417A | + | + | ++ | +++ |
| V234A+Y89A | + | +++ | ++ | ++ |
| V234A+N151A | ++ | 0 | ++ | ++ |
| V234A+L379A+L380A | ++ | 0 | ++ | + |
| V234A+D416A+S417A | + | ++ | + | ++ |
| V234A+D416A+S424A | + | 0 | + | ++ |
| V234A+S417A+ S424A | ++ | + | ++ | + |

| | | | | |
|---|---|---|---|---|
| Note: In the above table, 0 represented that the conversion rate was <1%, + represented that the conversion rate was greater than or equal to 1% and less than 5%, ++ represented that the conversion rate was greater than or equal to 5% and less than or equal to 10%, +++ represented that the conversion rate was greater than or equal to 10% and less than 15%, and ++++ represented that the conversion rate was greater than or equal to 15% and less than 20%. | | | | |

### Embodiment 3

On the basis of Embodiment 2, a plurality of rounds of a saturation mutation was performed, and the catalytic activity of the mutant was detected according to the following reaction conditions:
A 1 mL reaction system included 4 mg of Substrate 1 or Substrate 2 or Substrate 3 or Substrate 4, 1 mg of PLP, 2 mg of isopropylamine hydrochloride, 400 µL of crude enzyme solution (prepared from 200 mg of wet bacterial sludge), and pH 8.0 100 mM phosphate buffer, and it was reacted at 50°C for 18 h. Results were shown in Table 3.

**Table 3:**

| Mutation site | Conversion rate | | | |
|---|---|---|---|---|
| | Substrate 1 | Substrate 2 | Substrate 3 | Substrate 4 |
| V234A+L380A | ++ | + | ++ | ++ |
| V234A+L380A+Y89D | ++ | ++ | ++ | +++ |
| V234A+L380A+Y89S | ++ | ++ | ++ | +++ |
| V234A+L380A+Y89H | ++ | ++ | ++ | +++ |
| V234A+L380A+Y89M | ++ | +++ | +++ | +++ |
| V234A+L380A+F409A | +++ | ++ | ++ | +++ |
| V234A+L380A+F409S | ++ | + | ++ | +++ |
| V234A+L380A+F409Q | +++ | +++ | ++ | +++ |
| V234A+L380A+F409L | ++ | ++ | ++ | +++ |
| V234A+L380A+S424F | ++++ | +++ | +++ | +++ |
| V234A+L380A+S424Q | ++ | ++++ | ++ | +++ |
| V234A+L380A+S424P | +++ | ++ | ++ | +++ |
| V234A+L380A+S424R | ++++ | +++ | +++ | +++ |
| V234A+L380A+S424N | ++ | + | ++ | +++ |
| V234A+L380A+N86M | +++ | ++ | ++ | +++ |
| V234A+L380A+N86P | ++++ | ++ | +++ | +++ |
| V234A+L380A+N86D | ++ | +++ | ++ | +++ |
| V234A+L380A+N86A | ++ | ++ | ++ | +++ |
| V234A+L380A+N86V | +++ | ++ | ++ | +++ |
| V234A+L380A+N86H | +++ | +++ | +++ | +++ |
| V234A+L380A+N86M+Y89D | ++++ | +++ | +++ | ++++ |
| V234A+L380A+N86M+Y89S | +++ | +++ | +++ | ++++ |
| V234A+L380A+N86M+Y89G | +++ | +++ | +++ | ++++ |
| V234A+L380A+N86M+Y89H | +++ | +++ | +++ | ++++ |
| V234A+L380A+N86M+Y89F | +++ | +++ | +++ | ++++ |
| V234A+L380A+N86M+Y89A | ++++ | ++++ | +++ | ++++ |
| V234A+L380A+Y89D+F409H | +++ | +++++ | +++ | +++ |
| V234A+L380A+Y89D+F409A | +++ | ++ | +++ | ++++ |
| V234A+L380A+Y89D+F409P | ++++ | +++ | +++ | ++++ |
| V234A+L380A+Y89D+F409K | +++ | +++ | +++ | ++++ |
| V234A+L380A+Y89D+F409G | +++ | ++ | + | ++++ |
| V234A+L380A+Y89D+S424A | ++++ | +++ | +++++ | +++ |
| V234A+L380A+Y89D+S424V | ++++ | + | ++ | ++++ |
| V234A+L380A+Y89D+S424Y | +++ | +++ | +++ | ++++ |
| V234A+L380A+Y89D+S424E | ++++ | +++ | +++ | ++++ |
| V234A+L380A+Y89D+S424Q | +++ | +++ | +++ | ++++ |
| V234A+L380A+Y89D+N86H | ++++ | +++ | +++ | +++ |
| V234A+L380A+Y89D+N86A | ++++ | +++ | +++ | +++ |
| V234A+L380A+Y89D+N86H+Y85M | ++++ | +++ | +++ | ++++ |
| V234A+L380A+Y89D+N86H+Y85F | ++++ | ++++ | +++ | ++++ |
| V234A+L380A+Y89D+N86H+Y85R | ++++ | +++ | +++ | ++++ |
| V234A+L380A+Y89D+N86H+Y85M+S417Q | ++++ | +++ | ++++ | +++++ |
| V234A+L380A+Y89D+N86H+Y85M+S417F | ++++ | +++ | ++++ | ++++ |
| V234A+L380A+Y89D+N86H+Y85M+S417I | ++++ | +++ | +++ | +++++ |
| V234A+L380A+Y89D+N86H+Y85M+S424I | ++++ | ++++ | +++ | +++++ |
| V234A+L380A+Y89D+N86H+Y85M+P83C | ++++ | ++++ | +++ | +++++ |
| V234A+L380A+Y89D+N86H+Y85M+P83A | ++++ | +++ | +++ | +++++ |
| V234A+L380A+Y89D+N86H+Y85M+P83S | ++++ | +++ | ++++ | ++++ |
| V234A+L380A+Y89D+N86H+Y85M+P83G | ++++ | +++++ | +++ | +++++ |
| V234A+L380A+Y89D+N86H+Y85M+T91M | ++++ | ++++ | ++++ | ++++ |
| V234A+L380A+Y89D+N86H+Y85M+T91A | ++++ | ++++ | +++ | +++++ |
| V234A+L380A+Y89D+N86H+Y85M+T91V | ++++ | +++ | +++ | +++++ |
| V234A+L380A+Y89D+N86H+Y85M+T91N | ++++ | ++++ | ++++ | ++++ |
| V234A+L380A+Y89D+N86H+Y85M+T91I | ++++ | +++ | +++ | +++++ |
| V234A+L380A+Y89D+N86H+Y85M+T91M+P83S | ++++ | ++++ | ++++ | +++++ |
| V234A+L380A+Y89D+N86H+Y85M+T91M+P83C | ++++ | +++ | +++ | +++++ |
| V234A+L380A+Y89D+N86H+Y85M+T91M+P83A | ++++ | ++++ | +++ | +++++ |
| V234A+L380A+Y89D+N86H+Y85M+T91M+P83G | ++++ | +++ | +++ | +++++ |
| V234A+L380A+Y89D+N86H+Y85M+T91M+P83R | ++++ | ++++ | +++ | +++++ |
| V234A+L380A+Y89D+N86H+Y85M+T91M+P83S+K90Y | +++++ | +++++ | +++++ | ++++++ |
| V234A+L380A+Y89D+N86H+Y85M+T91M+P83S+K90G | +++++ | +++++ | +++++ | ++++++ |
| V234A+L380A+Y89D+N86H+Y85M+T91M+P83S+K90A | +++++ | +++++ | +++++ | ++++++ |
| V234A+L380A+Y89D+N86H+Y85M+T91M+P83S+K90V | +++++ | +++++ | +++++ | ++++++ |
| V234A+L380A+Y89D+N86H+Y85M+T91M+P83S+K90S | +++++ | ++++ | +++++ | +++++ |

| | | | | |
|---|---|---|---|---|
| Note: In the above table, 0 represented that the conversion rate was <1%, + represented that the conversion rate was greater than or equal to 1% and less than 5%, ++ represented that the conversion rate was greater than or equal to 5% and less than 10%, +++ represented that the conversion rate was greater than or equal to 10% and less than 15%, ++++ represented that the conversion rate was greater than or equal to 15% and less than 20%, +++++ represented that the conversion rate was greater than or equal to 20% and less than 40%, and ++++++ represented that the conversion rate was greater than or equal to 40%. | | | | |

### Embodiment 4

Some mutants in Embodiments 1, 2, and 3 were selected, and the tolerance of the mutants was detected according to the following reaction conditions:
A 1 mL reaction system included 4 mg of Substrate 1 or Substrate 2 or Substrate 3 or Substrate 4, 1 mg of PLP, 2 mg of isopropylamine hydrochloride, 400 µL of crude enzyme solution (prepared from 200 mg of wet bacterial sludge) after treatment at 70°C for 1 h, and pH 8.0 100 mM phosphate buffer, and it was reacted at 50°C for 18 h. Results were shown in Table 4.

**Table 4:**

| Mutation site | Relative residual activity | | | |
|---|---|---|---|---|
| | Substrate 1 | Substrate 2 | Substrate 3 | Substrate 4 |
| Female parent | + | + | + | + |
| V234A+D416A | + | + | + | + |
| V234A+S417A | ++ | ++ | ++ | ++ |
| V234A+L380A | ++ | ++ | ++ | ++ |
| V234A+L380A+Y89D+N86H | ++ | ++ | ++ | ++ |
| V234A+L380A+Y89D+N86A | ++ | ++ | ++ | ++ |
| V234A+L380A+Y89D+N86H+Y85M+P83S | ++ | ++ | ++ | ++ |
| V234A+L380A+Y89D+N86H+Y85M+P83G | ++ | ++ | ++ | ++ |
| V234A+L380A+Y89D+N86H+Y85M+T91M | ++ | ++ | ++ | ++ |
| V234A+L380A+Y89D+N86H+Y85M+T91M+P83S | +++ | +++ | +++ | +++ |
| V234A+L380A+Y89D+N86H+Y85M+T91M+P83C | +++ | +++ | +++ | +++ |
| V234A+L380A+Y89D+N86H+Y85M+T91M+P83S+K90Y | ++++ | ++++ | ++++ | ++++ |
| V234A+L380A+Y89D+N86H+Y85M+T91M+P83S+K90G | ++++ | +++ | ++++ | ++++ |
| V234A+L380A+Y89D+N86H+Y85M+T91M+P83S+K90A | ++++ | ++++ | ++++ | ++++ |

| | | | | |
|---|---|---|---|---|
| Note: In the above table, + represented that the relative residual activity was greater than or equal to 10% and less than 30%, ++ represented that the relative residual activity was greater than or equal to 30% and less than 40%, +++ represented that the relative residual activity was greater than or equal to 40% and less than 50%, and ++++ represented that the relative residual activity was greater than or equal to 50% and less than 60%. | | | | |

The relative residual activity refers to the enzyme activity measured in the enzyme solution after being subjected to extreme conditions such as high temperature, alkalinity, organic solvents, etc., expressed as a percentage of the enzyme activity under optimal conditions without exposure to extreme environments. Under the same treatment conditions, the relative residual activity was higher, it was indicated that the stability of the enzyme was higher under these conditions.

### Embodiment 5

On the basis of Embodiment 3, a plurality of rounds of error-prone PCR and combination mutation was performed, and the catalytic activity of the mutant was detected according to the following reaction conditions:
A 1 mL reaction system included 10 mg of Substrate 1 or Substrate 2 or Substrate 3 or Substrate 4, 1 mg of PLP, 2 mg of isopropylamine hydrochloride, 100 µL of crude enzyme solution (prepared from 50 mg of wet bacterial sludge), and pH 8.0 100 mM phosphate buffer, and it was reacted at 50°C for 18 h. Results were shown in Table 5.

**Table 5:**

| Mutation site | Conversion rate | | | |
|---|---|---|---|---|
| | Sub strat e 1 | Sub strat e 2 | Sub strat e 3 | Sub strat e 4 |
| V234A+L380A+Y89D+N86H+Y85M+T91M+P83S+K90Y | +++ | +++ | +++ | +++ |
| V234A+L380A+Y89D+N86H+Y85M+T91M+P83S+K90Y+S417I | +++ | +++ | +++ | +++ + |
| V234A+L380A+Y89D+N86H+Y85M+T91M+P83S+K90G+S417I | +++ | +++ | +++ | +++ |
| V234A+L380A+Y89D+N86H+Y85M+T91M+P83S+K90Y+S417A | +++ | +++ | +++ + | +++ + |
| V234C+L380A+Y89D+N86H+Y85M+T91M+P83S+K90Y+S417I | +++ | +++ | +++ | +++ + |
| V234A+L380A+Y89D+N86H+Y85M+T91M+P83S+K90Y+S417I+S424 A | +++ + | +++ + | +++ | +++ + |
| V234A+L380A+Y89D+N86H+Y85M+T91M+P83S+K90G+S417I+S424 A | +++ | +++ | +++ | +++ + |
| V234C+L380A+Y89D+N86H+Y85M+T91M+P83S+K90Y+S417I+S424 A | +++ | +++ | +++ | +++ + |
| V234A+L380A+Y89D+N86H+Y85M+T91M+P83S+K90Y+S417I+L379 S | +++ | +++ | +++ | +++ + |
| V234A+L380A+Y89D+N86H+Y85M+T91M+P83S+K90Y+S417I+S424 A+F301S | +++ | ++ | +++ | +++ + |
| V234A+L380A+Y89D+N86H+Y85M+T91M+P83S+K90G+S417I+S424 A+F301S | +++ | +++ | +++ | +++ + |
| V234A+L380A+Y89D+N86H+Y85M+T91M+P83S+K90Y+S417I+L379 S+H274Y | +++ | +++ | +++ + | +++ + |
| V234A+L380A+Y89D+N86H+Y85M+T91M+P83S+K90Y+S417I+L379 S+A239S | +++ | +++ + | +++ + | +++ |
| V234A+L380A+Y89D+N86H+Y85M+T91M+P83S+K90Y+S417I+L379 S +H274Y+F409L | +++ | +++ ++ | +++ + | +++ ++ |
| V234A+L380A+Y89D+N86H+Y85M+T91M+P83S+K90Y+S417I+S424 A+G164S | +++ ++ | +++ ++ | +++ ++ | +++ ++ |
| V234A+L380A+Y89D+N86H+Y85M+T91M+P83S+K90Y+S417I+S424 A+F301S | +++ ++ | +++ + | +++ + | +++ ++ |
| V234A+L380A+Y89D+N86H+Y85M+T91M+P83S+K90Y+S417I+S424 A+T452S | +++ ++ | +++ ++ | +++ + | +++ ++ |
| V234A+L380A+Y89D+N86H+Y85M+T91M+P83S+K90Y+S417I+S424A+F301S+G164S | ++++++ | +++++ | +++++ | +++++ |
| V234A+L380A+Y89D+N86H+Y85M+T91 M+P83S+K90G+S417I+S424 A+F301S+G164S | +++ +++ | +++ + | +++ +++ | +++ ++ |
| V234A+L380A+Y89D+N86H+Y85M+T91M+P83S+K90Y+S417I+S424 A+F301S+G164S+T452S | +++ +++ | +++ +++ | +++ +++ | +++ +++ |
| V234A+L380A+Y89D+N86H+Y85M+T91 M+P83S+K90G+S417I+S424 A+F301S+G164S+T452S | +++ ++ | +++ ++ | +++ ++ | +++ ++ |
| V234A+L380A+Y89D+N86H+Y85M+T91M+P83S+K90Y+S417I+S424 A+F301S+G164S+T452S+M180V | +++ ++ | +++ ++ | +++ + | +++ ++ |
| V234A+L380A+Y89D+N86H+Y85M+T91M+P83S+K90G+S417I+S424 A+F301S+G164S+T452S+M180V | +++ ++ | +++ ++ | +++ ++ | +++ ++ |
| V234A+L380A+Y89D+N86H+Y85M+T91M+P83S+K90Y+S417I+S424 A+F301S+G164S+T452S+M180V+F449L | +++ +++ | +++ +++ | +++ +++ | +++ ++ |
| V234A+L380A+Y89D+N86H+Y85M+T91 M+P83S+K90G+S417I+S424 A+F301S+G164S+T452S+M180V+F449L | +++ ++ | +++ + | +++ ++ | +++ ++ |
| V234A+L380A+Y89D+N86H+Y85M+T91M+P83S+K90Y+S417I+S424 A+F301S+G164S+T452S+M180V+F449L+T452S | +++ ++ | +++ + | +++ ++ | +++ ++ |
| V234A+L380A+Y89D+N86H+Y85M+T91M+P83S+K90Y+S417I+S424 A+F301S+G164S+T452S+M180V+F449L+F320H | +++ +++ | +++ +++ | +++ ++ | +++ ++ |
| V234A+L380A+Y89D+N86H+Y85M+T91M+P83S+K90Y+S417I+S424 A+F301S+G164S+T452S+M180V+F449L+Y322T | +++ +++ | +++ + | +++ ++ | +++ ++ |
| V234A+L380A+Y89D+N86H+Y85M+T91M+P83S+K90Y+S417I+S424 A+F301S+G164S+T452S+M180V+F449L+F320H +Y322T | +++ +++ | +++ ++ | +++ ++ | +++ ++ |
| V234A+L380A+Y89D+N86H+Y85M+T91M+P83S+K90G+S417I+S424 A+F301S+G164S+T452S+M180V+F449L+F320H +Y322T | +++ + | +++ ++ | +++ +++ | +++ ++ |
| V234A+L380A+Y89D+N86H+Y85M+T91I+P83S+K90Y+S417I+S424A +F301S+G164S+T452S+M180V+F449L+F320H+Y322T | +++ +++ | +++ ++ | +++ ++ | +++ ++ |
| V234A+L380A+Y89D+N86H+Y85M+T91I+P83S+K90G+S417I+S424 A+F301S+G164S+T452S+M180V+F449L+F320H+Y322T | +++ ++ | +++ ++ | +++ + | +++ +++ |
| V234A+L380A+Y89D+N86H+Y85M+T91I+P83S+K90Y+S417I+S424A +F301S+G164S+T452S+M180V+F449L+F320H+Y322T+D315V | +++ +++ | +++ ++ | +++ ++ | +++ +++ |
| V234A+L380A+Y89D+N86H+Y85M+T91I+P83S+K90Y+S417I+S424A +F301S+G164S+T452S+M180V+F449L+F320H+Y322T+I311F | +++ + | +++ +++ | +++ ++ | +++ +++ |
| V234A+L380A+Y89D+N86H+Y85M+T91I+P83S+K90Y+S417I+S424A +F301S+G164S+T452S+M180V+F449L+F320H+Y322T+D315V+A31 V | +++ ++ | +++ +++ | +++ ++ | +++ +++ |

| | | | | |
|---|---|---|---|---|
| Note: In the above table, 0 represented that the conversion rate was <1%, + represented that the conversion rate was greater than or equal to 1% and less than 5%, ++ represented that the conversion rate was greater than or equal to 5% and less than 10%, +++ represented that the conversion rate was greater than or equal to 10% and less than 15%, ++++ represented that the conversion rate was greater than or equal to 15% and less than 20%, +++++ represented that the conversion rate was greater than or equal to 20% and less than 40%, and ++++++ represented that the conversion rate was greater than or equal to 40%. | | | | |

### Embodiment 6

On the basis of Embodiment 5, a plurality of rounds of saturation mutation and combination mutation was performed, and the catalytic activity of the mutant was detected according to the following reaction conditions:
A 1 mL reaction system included 50 mg of Substrate 1 or Substrate 2 or Substrate 3 or Substrate 4, 1 mg of PLP, 10 mg of isopropylamine hydrochloride, 50 µL of crude enzyme solution (prepared from 25 mg of wet bacterial sludge), and pH 8.0 100 mM phosphate buffer, and it was reacted at 45°C for 18 h. Results were shown in Table 6.

**Table 6:**

| Mutation site | Conversion rate | | | |
|---|---|---|---|---|
| | Su bst rat e 1 | Su bst rat e 1 | Su bst rat e 1 | Su bst rat e 1 |
| V234A+L380A+Y89D+N86H+Y85M+T91I+P83S+K90Y+S417I+S424A+F3 01S+G164S+T452S+M180V+F449L+F320H+Y322T+D315V+A31V | ++ + | ++ + | ++ + | ++ + |
| V234A+L380A+Y89D+N86H+Y85M+T91I+P83S+K90Y+S417I+S424A+F3 01S+G164S+T452S+M180V+F449L+F320H+Y322T+D315C+A31V | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+V327 S | ++ + | ++ ++ | ++ ++ | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q | ++ + | ++ ++ | ++ + | ++ ++ |
| V234A+L380A+Y89D+N86H+Y85M+T91I+P83S+K90Y+S417I+S424A+F3 | ++ | ++ | ++ | ++ |
| 01S+G164S+T452S+M180V+F449L+F320H+Y322T+D315R+A31V+L295 M | + | ++ | ++ | ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295F | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64A | ++ + | ++ ++ | ++ + | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64S | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S | ++ + | ++ ++ | ++ + | ++ ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409Q | ++ ++ | ++ ++ | ++ ++ | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409V | ++ ++ | ++ ++ | ++ + | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409N | ++ + | ++ ++ | ++ ++ | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409N+T402K | ++ ++ | ++ ++ | ++ ++ + | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409V+T402S | ++ ++ + | ++ ++ | ++ ++ + | ++ ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409V+T402S+T126C | ++ ++ + | ++ ++ ++ | ++ ++ ++ | ++ ++ + |
| V234A+L380A+Y89D+N86H+Y85M+T91I+P83S+K90Y+S417I+S424A+F3 01S+G164S+T452S+M180V+F449L+F320H+Y322T+D315M+A31V+L295 Q+V64M+H154S+F409V+T402S | ++ ++ ++ | ++ ++ + | ++ ++ + | ++ ++ ++ |
| V234A+L380A+Y89D+N86H+Y85M+T91I+P83S+K90Y+S417I+S424A+F3 01S+G164S+T452S+M180V+F449L+F320H+Y322T+D315V+A31V+L295Q +V64M+H154S+F409V+T402S | ++ ++ + | ++ ++ | ++ ++ + | ++ ++ ++ |
| V234A+L380A+Y89D+N86H+Y85M+T91I+P83S+K90G+S417I+S424A+F3 01S+G164S+T452S+M180V+F449L+F320H+Y322T+D315R+A31V+L295 Q+V64M+H154S+F409V+T402S+T126C | ++ ++ + | ++ ++ + | ++ ++ ++ | ++ ++ + |
| V234A+L380A+Y89D+N86H+Y85M+T91I+P83S+K90G+S417I+S424A+F3 01S+G164S+T452S+M180V+F449L+F320H+Y322T+D315M+A31V+L295 Q+V64M+H154S+F409V+T402S | ++ ++ + | ++ ++ + | ++ ++ + | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S | ++ ++ + | ++ ++ + | ++ ++ + | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C | ++ ++ + | ++ ++ ++ | ++ ++ + | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L | ++ ++ ++ | ++ ++ ++ | ++ ++ ++ | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L+R77Q | ++ ++ ++ | ++ ++ ++ | ++ ++ ++ | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L+N317Y | ++ ++ ++ | ++ ++ ++ | ++ ++ ++ | ++ ++ ++ |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L+A433T | ++ ++ ++ | ++ ++ ++ | ++ ++ ++ | ++ ++ ++ |

| | | | | |
|---|---|---|---|---|
| Note: In the above table, 0 represented that the conversion rate was <1%, + represented that the conversion rate was greater than or equal to 1% and less than 5%, ++ represented that the conversion rate was greater than or equal to 5% and less than 10%, +++ represented that the conversion rate was greater than or equal to 10% and less than 20%, ++++ represented that the conversion rate was greater than or equal to 20% and less than 40%, +++++ represented that the conversion rate was greater than or equal to 40% and less than 60%, and ++++++ represented that the conversion rate was greater than or equal to 60%. | | | | |

### Embodiment 7

Some mutants in Embodiments 5 and 6 were selected, and the tolerance of the mutants was detected according to the following reaction conditions:
A 1 mL reaction system included 10 mg of Substrate 1 or Substrate 2 or Substrate 3 or Substrate 4, 1 mg of PLP, 2 mg of isopropylamine hydrochloride, 100 µL of crude enzyme solution (prepared from 50 mg of wet bacterial sludge) after treatment at 70°C for 1 h, and pH 8.0 100 mM phosphate buffer, and it was reacted at 50°C for 18 h. Results were shown in Table 7.

**Table 7:**

| Mutation site | Relative residual activity | | | |
|---|---|---|---|---|
| | Su bst rat e 1 | Su bst rat e2 | Su bst rat e 3 | Su bst rat e4 |
| V234A+L380A+Y89D+N86H+Y85M+T91 M+P83S+K90Y | + | + | + | + |
| V234A+L380A+Y89D+N86H+Y85M+T91M+P83S+K90Y+S417I+L379S+H 274Y+F409L | + | + | + | + |
| V234A+L380A+Y89D+N86H+Y85M+T91M+P83S+K90Y+S417I+S424A+G 164S | + | + | + | + |
| V234A+L380A+Y89D+N86H+Y85M+T91I+P83S+K90Y+S417I+S424A+F3 01S+G164S+T452S+M180V+F449L+F320H+Y322T+D315V | ++ | ++ | ++ | ++ |
| V234A+L380A+Y89D+N86H+Y85M+T91I+P83S+K90Y+S417I+S424A+F3 01S+G164S+T452S+M180V+F449L+F320H+Y322T+I311F | ++ | ++ | ++ | ++ |
| V234A+L380A+Y89D+N86H+Y85M+T91I+P83S+K90Y+S417I+S424A+F3 01S+G164S+T452S+M180V+F449L+F320H+Y322T+D315V+A31V | ++ | ++ | ++ | ++ |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409V+T402S | ++ + | ++ + | ++ + | ++ + |
| V234A+L380A+Y89D+N86H+Y85M+T91I+P83S+K90Y+S417I+S424A+F3 01S+G164S+T452S+M180V+F449L+F320H+Y322T+D315R+A31V+L295 Q+V64M+H154S+F409V+T402S+T126C | ++ + | ++ + | ++ + | ++ + |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L | ++ + | ++ + | ++ + | ++ + |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64MH154S+F409V+T402S+T126C+F364L+ N317Y | ++ + | ++ + | ++ + | ++ + |
| V234A+L380A+Y89D+N86H+Y85M+T91I+P83S+K90G+S417I+S424A+F3 01S+G164S+T452S+M180V+F449L+F320H+Y322T+D315V+A31V+L295Q +V64M+H154S+F409V+T402S+T126C+F364L+A433T | ++ + | ++ + | ++ + | ++ + |

| | | | | |
|---|---|---|---|---|
| Note: In the above table, + represented that the relative residual activity was greater than or equal to 50% and less than 60%, ++ represented that the relative residual activity was greater than or equal to 60% and less than 70%, and +++ represented that the relative residual activity was greater than or equal to 70%. | | | | |

### Embodiment 8

50 mL of 100 mmol/L phosphate buffer solution (5 vol) and 20 mL of 5 mol/L isopropylamine hydrochloride solution (2 vol) were added to a 250 mL four-necked flask at a room temperature, and pH was adjusted to 8.5∼9.0. 0.1 g of pyridoxal phosphate (1wt%) and 10 g of 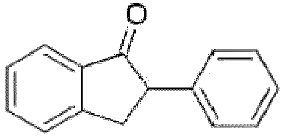 (Substrate 1) were added, and stirred uniformly, and then 2 mL (0.1wt, 0.5 g/mL) of enzyme solution of a transaminase mutant (V234A+L380A+Y89D+N86H+Y85M+T91I+P83S+K90G+S417I+S424A+F301S+G164S+T452S+ M180V+F449L+F320H+Y322T+D315V+A31V+L295Q+V64M+H154S+F409V+T402S+T126C+F36 4L) mutated on the basis of SEQ ID NO: 1 was added, and pH was adjusted to 8.5∼9.0. The temperature was raised to 45°C, and it was stirred and reacted overnight. After the reaction was completed, the system was acid-adjusted to pH=2-3, and the protein was denatured. A filtered filtrate was extracted with 50 mL of methyl tert-butyl ether. A water phase was adjusted to pH=12, and then it was extracted twice with 50 mL of the methyl tert-butyl ether. After a combined organic phase was dried with anhydrous magnesium sulfate, it was concentrated until there was no fraction under the conditions of T<40°C and P≤-0.06 Mpa. A target product 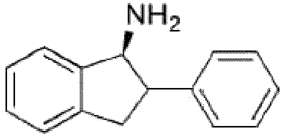 was obtained.

After being detected by HPLC, the purity was >99%, the de value was >99%, and the yield was 87%.

### Embodiment 9

50 mL of 100 mmol/L phosphate buffer solution (5 vol) and 20 mL of 5 mol/L isopropylamine hydrochloride solution (2 vol) were added to a 250 mL four-necked flask at a room temperature, and pH was adjusted to 8.5∼9.0. 0.1 g of pyridoxal phosphate (1wt%) and 10 g of 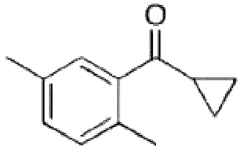 (Substrate 2) were added, and stirred uniformly, and then 2 mL (0.1wt, 0.5 g/mL) of enzyme solution of a transaminase mutant (V234A+L380A+Y89D+N86H+Y85M+T91I+P83S+K90G+S417I+S424A+F301S+G164S+T452S+ M180V+F449L+F320H+Y322T+D315V+A31V+L295Q+V64M+H154S+F409V+T402S+T126C+F36 4L) mutated on the basis of SEQ ID NO: 1 was added, and pH was adjusted to 8.5~9.0. The temperature was raised to 45°C, and it was stirred and reacted overnight. After the reaction was completed, the system was acid-adjusted to pH=2-3, and the protein was denatured. A filtered filtrate was extracted with 50 mL of methyl tert-butyl ether. A water phase was adjusted to pH=12, and then it was extracted twice with 50 mL of the methyl tert-butyl ether. After a combined organic phase was dried with anhydrous magnesium sulfate, it was concentrated until there was no fraction under the conditions of T<40°C and P≤-0.06 Mpa. A target product 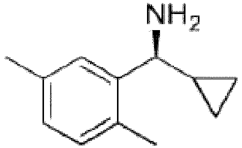 was obtained.

After being detected by HPLC, the purity was >98%, the de value was >99%, and the yield was 84%.

### Embodiment 10

50 mL of 100 mmol/L phosphate buffer solution (5 vol) and 20 mL of 5 mol/L isopropylamine hydrochloride solution (2 vol) were added to a 250 mL four-necked flask at a room temperature, and pH was adjusted to 8.5∼9.0. 0.1 g of pyridoxal phosphate (1wt%) and 10 g of 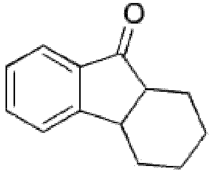 (Substrate 3) were added, and stirred uniformly, and then 2 mL (0.1wt, 0.5 g/mL) of enzyme solution of a CvTA transaminase mutant (V234A+L380A+Y89D+N86H+Y85M+T91I+P83S+K90G+S417I+S424A+F301S+G164S+T452S+ M180V+F449L+F320H+Y322T+D315V+A31V+L295Q+V64M+H154S+F409V+T402S+T126C+F36 4L) mutated on the basis of SEQ ID NO: 1 was added, and pH was adjusted to 8.5∼9.0. The temperature was raised to 45°C, and it was stirred and reacted overnight. After the reaction was completed, the system was acid-adjusted to pH=2-3, and the protein was denatured. A filtered filtrate was extracted with 50 mL of methyl tert-butyl ether. A water phase was adjusted to pH=12, and then it was extracted twice with 50 mL of the methyl tert-butyl ether. After a combined organic phase was dried with anhydrous magnesium sulfate, it was concentrated until there was no fraction under the conditions of T<40°C and P≤-0.06 Mpa. A target product 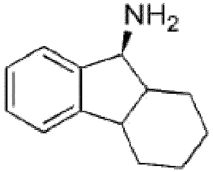 was obtained.

After being detected by HPLC, the purity was >98%, the de value was >99%, and the yield was 82%.

### Embodiment 11

50 mL of 100 mmol/L phosphate buffer solution (5 vol) and 20 mL of 5 mol/L isopropylamine hydrochloride solution (2 vol) were added to a 250 mL four-necked flask at a room temperature, and pH was adjusted to 8.5∼9.0. 0.1 g of pyridoxal phosphate (1wt%) and 10 g of 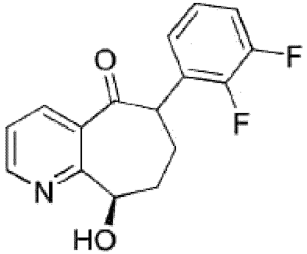 (Substrate 4) were added, and stirred uniformly, and then 2 mL (0.1wt, 0.5 g/mL) of enzyme solution of a transaminase mutant (V234A+L380A+Y89D+N86H+Y85M+T91I+P83S+K90G+S417I+S424A+F301S+G164S+T452S+ M180V+F449L+F320H+Y322T+D315V+A31V+L295Q+V64M+H154S+F409V+T402S+T126C+F36 4L) mutated on the basis of SEQ ID NO: 1 was added, and pH was adjusted to 8.5∼9.0. The temperature was raised to 45°C, and it was stirred and reacted overnight. After the reaction was completed, the system was acid-adjusted to pH=2-3, and the protein was denatured. A filtered filtrate was extracted with 50 mL of methyl tert-butyl ether. A water phase was adjusted to pH=12, and then it was extracted twice with 50 mL of the methyl tert-butyl ether. After a combined organic phase was dried with anhydrous magnesium sulfate, it was concentrated until there was no fraction under the conditions of T<40°C and P≤-0.06 Mpa. A target product 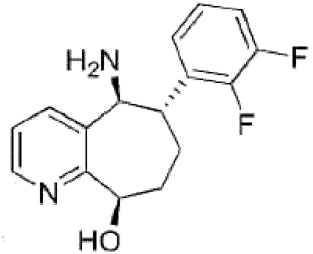 was obtained.

After being detected by HPLC, the purity was >99%, the de value was >99%, and the yield was 91%.

### Embodiment 12

Enzyme solution of a transaminase mutant (V234A+L380A+Y89D+N86H+Y85M+T91I+P83S+K90G+S417I+S424A+F301S+G164S+T452S+ M180V+F449L+F320H+Y322T+D315V+A31V+L295Q+V64M+H154S+F409V+T402S+T126C+F36 4) mutated on the basis of SEQ ID NO: 1 was used, and a catalytic reaction was performed on Substrates 5-16 by referring to the catalytic synthesis steps of Embodiments 6 to 9. Results were shown in Table 8:

**Table 8:**

| Substrate | Purity | de value | Yield |
|---|---|---|---|
| 5 | >99% | >99% | 87% |
| 6 | >98% | >99% | 87% |
| 7 | >98% | >99% | 82% |
| 8 | >99% | >99% | 90% |
| 9 | >98% | >99% | 86% |
| 10 | >99% | >99% | 84% |
| 11 | >99% | >99% | 89% |
| 12 | >99% | >99% | 85% |
| 13 | >98% | >99% | 86% |
| 14 | >99% | >99% | 90% |
| 15 | >99% | >99% | 86% |
| 16 | >99% | >99% | 83% |

From the above description, it may be seen that the embodiments of the present disclosure achieve the following technical effects: based on the obtained transaminase female parent with the excellent activity in the present disclosure, a plurality of sites that may improve the transaminase activity and/or tolerance is discovered by exploring the active sites of the transaminases, and by exploring the combinations of one or more sites, various transaminase mutants with excellent performance are obtained, and the above transaminase mutants have advantages such as wide substrate spectrum, ability to catalyze the synthesis of large sterically hindered chiral amine compounds, high enzyme activity, and strong tolerance to extreme environments, which may meet the needs of process production.

The above are only preferred embodiments of the present disclosure and are not intended to limit the present disclosure. For those skilled in the art, the present disclosure may have various modifications and variations. Any modifications, equivalent replacements, improvements and the like made within the spirit and principles of the present disclosure shall be contained within the scope of protection of the present disclosure.

## Claims

1. A transaminase mutant, comprising:
(a) a protein having an amino acid sequence shown in SEQ ID NO: 1;
(b) a protein having an amino acid sequence shown in SEQ ID NO: 2 or SEQ ID NO: 3;
(c) a protein having an amino acid mutation in at least one of the following sites of the amino acid sequence in (b): Y89, L380, N86, Y85, T91, P83, K90, S417, S424, F301, G164, T452, M180, F449, F320, Y322, D315, A31, L295, V64, H154, F409, T402, T126, F364, A433, L379, D416, N151, H274, I311, V327, R77 or N317, and having a transaminase function; or
(d) a protein having more than 80% of the homology with the amino acid sequence defined in any one of (a), (b) or (c) and having a transaminase function.

2. The transaminase mutant according to claim 1, wherein the amino acid mutations of the (c) are each independently selected from the following:
S424A or S424F or S424Q or S424P or S424R or S424N or S424V or S424Y or S424E or S424I;
L380A;
S417A or S417Q or S417F or S417I;
Y89A or Y89D or Y89S or Y89H or Y89M or Y89G or Y89F;
F409A or F409S or F409Q or F409L or F409H or F409P or F409K or F409G or F409V or F409N;
N86M or N86P or N86D or N86A or N86V or N86H;
Y85M or Y85F or Y85R;
P83C or P83A or P83S or P83G or P83R;
T91M orT91A or T91V orT91N or T91I;
K90Y or K90G or K90A or K90V or K90S;
F301S;
G164S;
T452S;
M180V;
F449L;
F320H;
Y322T;
D315V or D315C or D315R or D315M;
A31V;
L295Q or L295M or L295F or L295Q;
V64M or V64A or V64S;
H154S;
T402K or T402S;
T126C;
F364L;
A433T;
L379A or L379S;
D416A;
N151A;
H274Y;
I311F;
V327S;
R77Q;
N317Y;
wherein, the letter before the number represents an original amino acid, and the letter after the number represents a mutant amino acid.

3. The transaminase mutant according to claim 2, wherein in the (d), the protein has more than 85% of the homology with the amino acid sequence defined in (a), (b) or (c) and has the transaminase function.

4. The transaminase mutant according to claim 2 or 3, wherein the mutation of the transaminase mutant comprises any one of the following amino acid mutations:
W60A; Y89A; N151A; F166A; E168A; V234A; I262A; L379A; L380A; R405A; D416A; S417A;
C418A; S424A; V234A+S424A; V234A+L380A; V234A+ L379A; V234A+D416A; V234A+S417A; V234A+Y89A; V234A+N151A;
V234A+L379A+L380A; V234A+D416A+S417A; V234A+D416A+S424A; V234A+S417A+S424A; V234A+L380A+Y89D; V234A+L380A+Y89S; V234A+L380A+Y89H; V234A+L380A+Y89M; V234A+L380A+F409A; V234A+L380A+F409S; V234A+L380A+F409Q; V234A+L380A+F409L; V234A+L380A+S424F; V234A+L380A+S424Q; V234A+L380A+S424P; V234A+L380A+S424R; V234A+L380A+S424N; V234A+L380A+N86M; V234A+L380A+N86P; V234A+L380A+N86D; V234A+L380A+N86A; V234A+L380A+N86V; V234A+L380A+N86H;
V234A+L380A+N86M+Y89D; V234A+L380A+N86M+Y89S; V234A+L380A+N86M+Y89G; V234A+L380A+N86M+Y89H; V234A+L380A+N86M+Y89F; V234A+L380A+N86M+Y89A; V234A+L380A+Y89D+F409H; V234A+L380A+Y89D+F409A; V234A+L380A+Y89D+F409P; V234A+L380A+Y89D+F409K; V234A+L380A+Y89D+F409G; V234A+L380A+Y89D+S424A; V234A+L380A+Y89D+S424V; V234A+L380A+Y89D+S424Y; V234A+L380A+Y89D+S424E; V234A+L380A+Y89D+S424Q; V234A+L380A+Y89D+N86H; V234A+L380A+Y89D+N86A;
V234A+L380A+Y89D+N86H+Y85M;
V234A+L380A+Y89D+N86H+Y85F;
V234A+L380A+Y89D+N86H+Y85R;
V234A+L380A+Y89D+N86H+Y85M+S417Q;
V234A+L380A+Y89D+N86H+Y85M+S417F;
V234A+L380A+Y89D+N86H+Y85M+S417I;
V234A+L380A+Y89D+N86H+Y85M+S424I;
V234A+L380A+Y89D+N86H+Y85M+P83C;
V234A+L380A+Y89D+N86H+Y85M+P83A;
V234A+L380A+Y89D+N86H+Y85M+P83S;
V234A+L380A+Y89D+N86H+Y85M+P83G;
V234A+L380A+Y89D+N86H+Y85M+T91M;
V234A+L380A+Y89D+N86H+Y85M+T91A;
V234A+L380A+Y89D+N86H+Y85M+T91V;
V234A+L380A+Y89D+N86H+Y85M+T91N;
V234A+L380A+Y89D+N86H+Y85M+T91I;
V234A+L380A+Y89D+N86H+Y85M+T91 M+P83S;
V234A+L380A+Y89D+N86H+Y85M+T91 M+P83C;
V234A+L380A+Y89D+N86H+Y85M+T91M+P83A;
V234A+L380A+Y89D+N86H+Y85M+T91M+P83G;
V234A+L380A+Y89D+N86H+Y85M+T91M+P83R;
V234A+L380A+Y89D+N86H+Y85M+T91M+P83S+K90Y;
V234A+L380A+Y89D+N86H+Y85M+T91M+P83S+K90G;
V234A+L380A+Y89D+N86H+Y85M+T91M+P83S+K90A;
V234A+L380A+Y89D+N86H+Y85M+T91M+P83S+K90V;
V234A+L380A+Y89D+N86H+Y85M+T91 M+P83S+K90S;
V234A+L380A+Y89D+N86H+Y85M+T91M+P83S+K90Y+S417I;
V234A+L380A+Y89D+N86H+Y85M+T91M+P83S+K90G+S417I;
V234A+L380A+Y89D+N86H+Y85M+T91M+P83S+K90Y+S417A;
V234C+L380A+Y89D+N86H+Y85M+T91M+P83S+K90Y+S417I;
V234A+L380A+Y89D+N86H+Y85M+T91M+P83S+K90Y+S417I+S424A;
V234A+L380A+Y89D+N86H+Y85M+T91 M+P83S+K90G+S417I+S424A;
V234C+L380A+Y89D+N86H+Y85M+T91M+P83S+K90Y+S417I+S424A;
V234A+L380A+Y89D+N86H+Y85M+T91M+P83S+K90Y+S4171+L379S;
V234A+L380A+Y89D+N86H+Y85M+T91M+P83S+K90Y+S4171+S424A+F301S;
V234A+L380A+Y89D+N86H+Y85M+T91M+P83S+K90G+S417I+S424A+F301S;
V234A+L380A+Y89D+N86H+Y85M+T91M+P83S+K90Y+S417I+L379S+H274Y;
V234A+L380A+Y89D+N86H+Y85M+T91M+P83S+K90Y+S417I+L379S+A239S;
V234A+L380A+Y89D+N86H+Y85M+T91M+P83S+K90Y+S417I+L379S +H274Y+F409L;
V234A+L380A+Y89D+N86H+Y85M+T91 M+P83S+K90Y+S417I+S424A+G164S;
V234A+L380A+Y89D+N86H+Y85M+T91M+P83S+K90Y+S417I+S424A+T452S;
V234A+L380A+Y89D+N86H+Y85M+T91M+P83S+K90Y+S4171+S424A+F301S+G164S;
V234A+L380A+Y89D+N86H+Y85M+T91M+P83S+K90G+S417I+S424A+F301S+G164S;
V234A+L380A+Y89D+N86H+Y85M+T91M+P83S+K90Y+S4171+S424A+F301S+G164S+T45 2S;
V234A+L380A+Y89D+N86H+Y85M+T91M+P83S+K90G+S417I+S424A+F301S+G164S+T45 2S;
V234A+L380A+Y89D+N86H+Y85M+T91M+P83S+K90Y+S4171+S424A+F301S+G164S+T45 2S+M180V;
V234A+L380A+Y89D+N86H+Y85M+T91M+P83S+K90G+S417I+S424A+F301S+G164S+T45 2S+M180V;
V234A+L380A+Y89D+N86H+Y85M+T91M+P83S+K90Y+S4171+S424A+F301S+G164S+T45 2S+M180V+F449L;
V234A+L380A+Y89D+N86H+Y85M+T91M+P83S+K90G+S417I+S424A+F301S+G164S+T45 2S+M180V+F449L;
V234A+L380A+Y89D+N86H+Y85M+T91M+P83S+K90Y+S4171+S424A+F301S+G164S+T45 2S+M180V+F449L+T452S;
V234A+L380A+Y89D+N86H+Y85M+T91M+P83S+K90Y+S4171+S424A+F301S+G164S+T45 2S+M180V+F449L+F320H;
V234A+L380A+Y89D+N86H+Y85M+T91M+P83S+K90Y+S4171+S424A+F301S+G164S+T45 2S+M180V+F449L+Y322T;
V234A+L380A+Y89D+N86H+Y85M+T91M+P83S+K90Y+S4171+S424A+F301S+G164S+T45 2S+M180V+F449L+F320H +Y322T;
V234A+L380A+Y89D+N86H+Y85M+T91M+P83S+K90G+S417I+S424A+F301S+G164S+T45 2S+M180V+F449L+F320H +Y322T;
V234A+L380A+Y89D+N86H+Y85M+T91I+P83S+K90Y+S417I+S424A+F301S+G164S+T452 S+M180V+F449L+F320H+Y322T;
V234A+L380A+Y89D+N86H+Y85M+T91I+P83S+K90G+S417I+S424A+F301S+G164S+T452 S+M180V+F449L+F320H+Y322T;
V234A+L380A+Y89D+N86H+Y85M+T91I+P83S+K90Y+S417I+S424A+F301S+G164S+T452 S+M180V+F449L+F320H+Y322T+D315V;
V234A+L380A+Y89D+N86H+Y85M+T91I+P83S+K90Y+S417I+S424A+F301S+G164S+T452 S+M180V+F449L+F320H+Y322T+I311F;
V234A+L380A+Y89D+N86H+Y85M+T91I+P83S+K90Y+S417I+S424A+F301S+G164S+T452 S+M180V+F449L+F320H+Y322T+D315V+A31V;
V234A+L380A+Y89D+N86H+Y85M+T91I+P83S+K90Y+S417I+S424A+F301S+G164S+T452 S+M180V+F449L+F320H+Y322T+D315C+A31V;
V234A+L380A+Y89D+N86H+Y85M+T91I+P83S+K90Y+S417I+S424A+F301S+G164S+T452 S+M180V+F449L+F320H+Y322T+D315R+A31V;
V234A+L380A+Y89D+N86H+Y85M+T91I+P83S+K90Y+S417I+S424A+F301S+G164S+T452 S+M180V+F449L+F320H+Y322T+D315R+A31V+V327S;
V234A+L380A+Y89D+N86H+Y85M+T91I+P83S+K90Y+S417I+S424A+F301S+G164S+T452 S+M180V+F449L+F320H+Y322T+D315R+A31V+L295Q;
V234A+L380A+Y89D+N86H+Y85M+T91I+P83S+K90Y+S417I+S424A+F301S+G164S+T452 S+M180V+F449L+F320H+Y322T+D315R+A31V+L295M;
V234A+L380A+Y89D+N86H+Y85M+T91I+P83S+K90Y+S417I+S424A+F301S+G164S+T452 S+M180V+F449L+F320H+Y322T+D315R+A31V+L295F;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64A;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409Q;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409N;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409N+T402K;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V+T402S;
V234A+L380A+Y89D+N86H+Y85M+T91I+P83S+K90Y+S417I+S424A+F301S+G164S+T452 S+M180V+F449L+F320H+Y322T+D315R+A31V+L295Q+V64M+H154S+F409V+T402S+T12 6C;
V234A+L380A+Y89D+N86H+Y85M+T91I+P83S+K90Y+S417I+S424A+F301S+G164S+T452 S+M180V+F449L+F320H+Y322T+D315M+A31V+L295Q+V64M+H154S+F409V+T402S;
V234A+L380A+Y89D+N86H+Y85M+T91I+P83S+K90Y+S417I+S424A+F301S+G164S+T452 S+M180V+F449L+F320H+Y322T+D315V+A31V+L295Q+V64M+H154S+F409V+T402S;
V234A+L380A+Y89D+N86H+Y85M+T91 I+P83S+K90G+S417I+S424A+F301S+G164S+T452 S+M180V+F449L+F320H+Y322T+D315R+A31V+L295Q+V64M+H154S+F409V+T402S+T12 6C;
V234A+L380A+Y89D+N86H+Y85M+T91 I+P83S+K90G+S417I+S424A+F301S+G164S+T452 S+M180V+F449L+F320H+Y322T+D315M+A31V+L295Q+V64M+H154S+F409V+T402S;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S+T12 6C;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S+T12 6C+F364L;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S+T12 6C+F364L+R77Q;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S+T12 6C+F364L+N317Y;
V234A+L380A+Y89D+N86H+Y85M+T91 I+P83S+K90G+S417I+S424A+F301S+G164S+T452 S+M180V+F449L+F320H+Y322T+D315V+A31V+L295Q+V64M+H154S+F409V+T402S+T12 6C+F364L+A433T; and
V234A+L380A+Y89D+N86H+Y85M+T91 I+P83S+K90G+S417I+S424A+F301S+G164S+T452 S+M180V+F449L+F320H+Y322T+D315V+A31V+L295Q+V64MH154S+F409V+T402S+T126 C+F364L+N317Y.

5. A deoxyribonucleic acid (DNA) molecule, wherein the DNA molecule encodes the transaminase mutant according to any one of claims 1 to 4.

6. A recombinant plasmid, wherein the recombinant plasmid is linked with the DNA molecule according to claim 5.

7. A host cell, wherein the recombinant plasmid according to claim 6 is transformed in the host cell.

8. The host cell according to claim 7, wherein the host cell comprises a prokaryotic cell.

9. The host cell according to claim 8, wherein the prokaryotic cell comprises *Escherichia coli.*

10. A method for preparing a chiral amine compound, wherein the method comprises using the transaminase mutant according to any one of claims 1 to 4 under the action of an amino donor, and performing a transamination reaction on a ketone substrate shown in Formula I, to prepare the chiral amine compound, 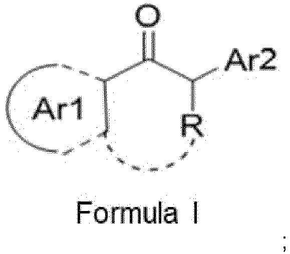
Ar1 is selected from a first substituted aryl, a first unsubstituted aryl, substituted arylene or unsubstituted arylene, substituted heteroarylene or unsubstituted heteroarylene;
Ar2 is selected from a second substituted aryl, a second unsubstituted aryl, a substituted cycloalkyl, an unsubstituted cycloalkyl, an alkyl or an alkylene;
R is selected from H, an alkyl, an alkylene or an alkylidene, the number of C atoms of the alkylene or alkylidene is selected from 1-5, and the alkylene or alkylidene comprises a substituted alkylene or alkylidene or an unsubstituted alkylene or alkylidene;
while R is selected from the alkylene or alkylidene, the alkylene or alkylidene is linked with the Ar1 and/or the Ar2 to form a ring;
substituents in the first substituted aryl, the substituted arylene, the substituted heteroarylene, the second substituted aryl or the substituted alkylene or alkylidene are each independently selected from a halogen, a hydroxyl, an amino, a methyl, an ethyl or -CH₂CH₂OH; and
heteroatoms in the substituted heteroarylene are selected from N, O or S.

11. The method according to claim 10, wherein the substituents of the first substituted aryl, the second substituted aryl or the substituted arylene are each independently selected from the halogen or the -CH₂CH₂OH.

12. The method according to claim 11, wherein the substituents are each independently located at any one or more of an ortho position, a meta position or a para position of the first substituted aryl, the second substituted aryl or the substituted arylene.

13. The method according to claim 12, wherein the halogen is selected from F, Cl or Br.

14. The method according to claim 13, wherein,
the Ar1 is selected from the first substituted aryl, the substituted arylene, the first unsubstituted aryl or the unsubstituted arylene,
the Ar2 is selected from the second unsubstituted aryl,
the R is selected from the unsubstituted alkylene, and the number of C atoms of the unsubstituted alkylene is selected from 1-5; and
the unsubstituted alkylene is linked with the Ar1 to form a ring.

15. The method according to claim 13, wherein,
the Ar1 is selected from the unsubstituted heteroarylene,
the Ar2 is selected from the second substituted aryl, and the substituent of the second substituted aryl is selected from the halogen;
the R is selected from the substituted alkylene, the substituent of the substituted alkylene is selected from the hydroxyl, and the number of C atoms of the substituted alkylene is selected from 1-5; and
the substituted alkylene is linked with the Ar1 to form a ring.

16. The method according to claim 13, wherein,
the Ar1 is selected from the substituted arylene, and the substituent of the substituted arylene is the halogen;
the Ar2 is selected from the substituted cycloalkyl or the unsubstituted cycloalkyl, and the number of C atoms of the substituted cycloalkyl or the unsubstituted cycloalkyl is selected from 3-8; and
the R is selected from the H.

17. The method according to claim 13, wherein,
the Ar1 is selected from the first unsubstituted aryl;
the Ar2 is selected from the second substituted aryl, the second unsubstituted aryl, the substituted cycloalkyl or the unsubstituted cycloalkyl; and
the R is selected from the H, the methyl, a methylene or a methine.

18. The method according to claim 13, wherein,
the Ar1 is selected from the cycloalkyl or the first substituted aryl, and the substituent is selected from the hydroxyl, the methyl, the ethyl or the -CH₂CH₂OH;
the Ar2 is selected from the second unsubstituted aryl or the alkyl; and
the R is selected from the H, the methyl, the methylene or the methine.

19. The method according to any one of claims 10 to 18, wherein the ketone substrate is selected from

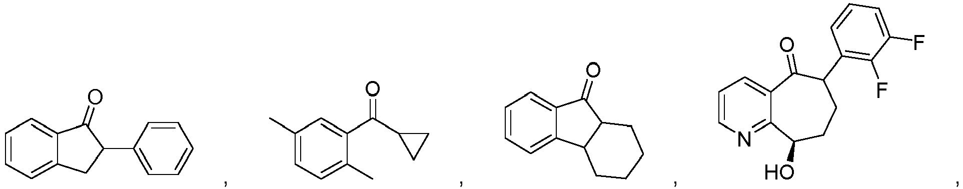

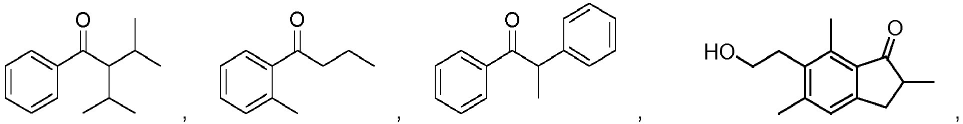

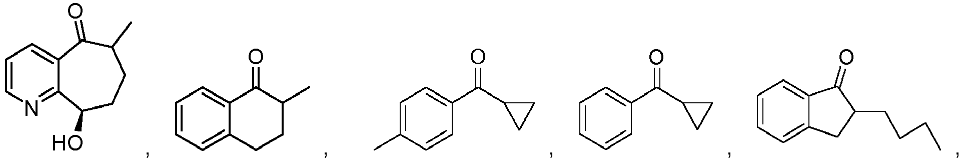

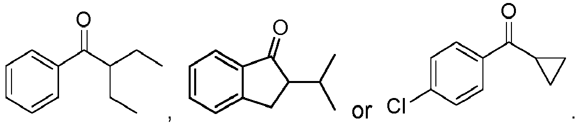

20. The method according to claim 19, wherein the amino donor comprises isopropylamine, isopropylamine hydrochloride, alanine, n-butylamine or aniline.
